(19) Europäisches Patentamt
European Patent Office
Office européen des brevets

(11) Veröffentlichungsnummer : **0 068 260**
**B1**

(12) # EUROPÄISCHE PATENTSCHRIFT

(45) Veröffentlichungstag der Patentschrift : **05.03.86**

(21) Anmeldenummer : **82105172.9**

(22) Anmeldetag : **14.06.82**

(51) Int. Cl.⁴ : **C 07 C 69/712**, C 07 D213/64, C 07 C 67/31, C 07 C 67/08, C 07 C 67/14, C 07 C 67/30, C 07 C 79/46, C 07 C121/50, A 01 N 43/40, A 01 N 39/02

(54) **Substituierte Phenoxypropionate, Verfahren zu ihrer Herstellung und ihre Verwendung als Herbizide.**

(30) Priorität : **25.06.81 JP 97486/81**
**16.09.81 JP 144778/81**
**07.12.81 JP 195604/81**
**07.12.81 JP 195605/81**

(43) Veröffentlichungstag der Anmeldung : **05.01.83 Patentblatt 83/01**

(45) Bekanntmachung des Hinweises auf die Patenterteilung : **05.03.86 Patentblatt 86/10**

(84) Benannte Vertragsstaaten :
**AT BE CH DE FR GB IT LI NL SE**

(56) Entgegenhaltungen :
**DE-A- 2 909 816**
**FR-A- 2 360 253**

(73) Patentinhaber : **NIHON TOKUSHU NOYAKU SEIZO K.K.**
**No.4, 2-Chome, Nihonbashi Honcho**
**Chuo-ku Tokyo 103 (JP)**

(72) Erfinder : **Aya, Masahiro**
**2-844, Suzuki-cho Kodaira-shi**
**Tokyo (JP)**
Erfinder : **Saito, Junichi**
**3-7-12, Osawa Mitaka-shi**
**Tokyo (JP)**
Erfinder : **Yasui, Kazuomi**
**7-6-15, Shakujiidai Nerima-ku**
**Tokyo (JP)**
Erfinder : **Shiokawa, Kozo**
**210-6, Shukugawara Tama-ku**
**Kawasaki-shi Kanagawa-ken (JP)**
Erfinder : **Moriya, Koichi**
**39-15, Namiki-cho Hachioji-shi**
**Tokyo (JP)**

(74) Vertreter : **Schumacher, Günter, Dr. et al**
**c/o Bayer AG Konzernverwaltung RP Patentabteilung**
**D-5090 Leverkusen 1 Bayerwerk (DE)**

**EP 0 068 260 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft bestimmte neue substituierte Phenoxypropionate, mehrere Verfahren zu deren Herstellung sowie deren Verwendung als Herbizide.

Die vorliegende Erfindung betrifft weiterhin neue Zwischenprodukte für die Herstellung der genannten Phenoxypropionate sowie ein Verfahren zur Herstellung dieser Zwischenprodukte.

In der US-PS 4 046 553 (entsprechend der JP-OS 51 106 735) wurde offenbart, daß Verbindungen der allgemeinen Formel

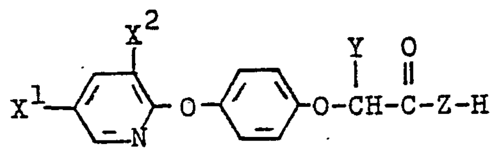

in der

$X^1$ und $X^2$ jeweils für Halogen,

Y für Wasserstoff oder Alkyl mit 6 oder weniger Kohlenstoff-Atomen und

Z für Sauerstoff oder Schwefel stehen,

und deren Salze, Ester, Amide und Halogenide herbizide Wirksamkeit besitzen.

In der JP-OS 52 131 545 (entsprechend der DE-PS 2 617 804) wurde offenbart, daß Verbindungen der allgemeinen Formel

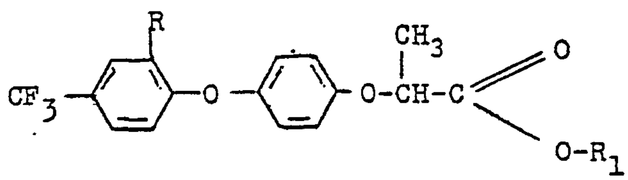

in der

R für Wasserstoff oder Halogen steht und

$R_1$ für

(a) ein geradkettiges oder kettenverzweigtes $C_1$-$C_{12}$-Alkyl (das durch Cyclohexyl, Halogenophenyl, Nitrophenyl, $C_1$-$C_6$-Alkylphenyl, Phenoxy, das in einigen Fällen durch Halogen und/oder $C_1$-$C_4$-Alkyl mono- bis tri-substituiert ist, $C_5$-$C_6$-Alkoxy, $C_5$-$C_6$-Alkoxy-$C_2$-$C_4$-alkoxy, $C_1$-$C_4$-Alkoxyethoxyethoxy, $C_1$-$C_4$-Acyl, eine durch die allgemeine Formel

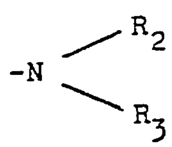 oder 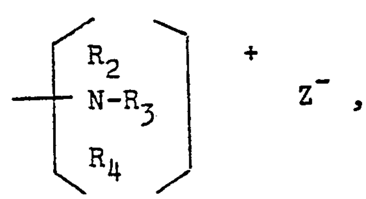

bezeichnete Gruppe substituiert oder in der 2-Stellung oder einer von der Carboxyl-Gruppe entfernten Stellung durch Phenyl mono- oder poly-substituiert ist);

(b) Cyclohexenyl oder Phenyl-$C_3$-$C_4$-alkenyl;

(c) $C_3$-$C_4$-Alkinyl (das gegebenenfalls durch geradkettiges oder kettenverzweigtes $C_1$-$C_4$-Alkyl, Halogen, Phenyl, Halogenophenyl oder $C_1$-$C_4$-Alkylphenyl mono- oder di-substituiert ist, jedoch mit der Maßgabe, daß $R_1$ nicht für unsubstituiertes Propargyl oder Butinyl steht);

(d) eine der durch die nachstehenden Formeln bezeichneten Gruppen

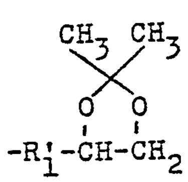 , $-R_1'-O-CO-R_5$ , 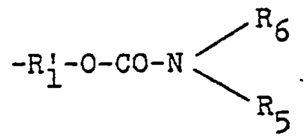 und

(IV') (V') (VI')

$-R_1'-S(O)_n-R_6$

(VII')

oder

(e) ein $C_1$-$C_2$-Alkyl, das durch Furyl, Tetrahydrofuryl, Pyridyl oder Oxiranyl substituiert ist, stehen,

herbizide Wirksamkeit besitzen,

wobei in den vorstehenden Formeln

$R_2$ Wasserstoff, $C_1$-$C_4$-Alkyl oder $C_1$-$C_4$-Alkoxy bezeichnet und

$R_3$ Wasserstoff, $C_1$-$C_4$-Alkyl oder Phenyl bezeichnet, oder

$R_2$ und $R_3$ zusammen eine 4- oder 5-gliedrige, gesättigte oder ungesättigte Alkylen-Kette bilden, in der ein Methylen gegebenenfalls durch —O—, $-\underset{\underset{O}{\|}}{C}-$ oder —N—($C_1$-$C_4$)-Alkyl ersetzt sein kann,

$R_4$ Wasserstoff oder $C_1$-$C_4$-Alkyl bezeichnet,

$Z^-$ für ein anorganisches oder organisches Anion steht,

$R_1'$ ein geradkettiges oder kettenverzweigtes $C_1$-$C_{12}$-Alkylen bezeichnet,

$R_5$ Wasserstoff, $C_1$-$C_4$-Alkyl, $C_1$-$C_4$-Halogenoalkyl, Phenyl, das gegebenenfalls durch Halogen, Nitro und/oder $C_1$-$C_4$-Alkyl substituiert ist, oder eine durch die Formeln

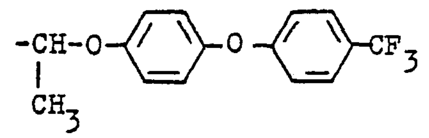

(VIII')

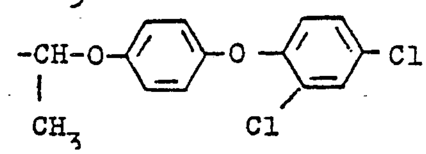

(IX')

dargestellte Gruppe bezeichnet,

$R_6$ für $C_1$-$C_4$-Alkyl steht und

n 0, 1 oder 2 ist.

In der JP-OS 52-144 637 (entsprechend der DE-PS 2 623 558) wurde offenbart, daß Verbindungen der allgemeinen Formel

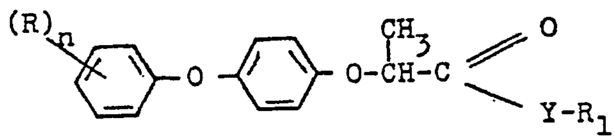

in der

R gleich oder verschieden sein können und jeweils für Halogen, $C_1$-$C_4$-Alkyl oder $C_1$-$C_4$-Alkoxy stehen,

Y O oder S bezeichnet,

n 1 oder 2 ist und

$R_1$ für

(a) ein geradkettiges oder kettenverzweigtes $C_1$-$C_{12}$-Alkyl (das durch Cyclohexyl, Halogenophenyl, Nitrophenyl, $C_1$-$C_4$-Alkylphenyl oder eine durch die allgemeine Formel

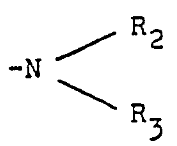 ; 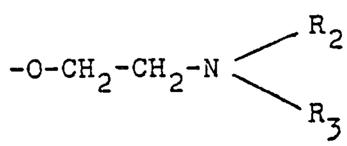 oder 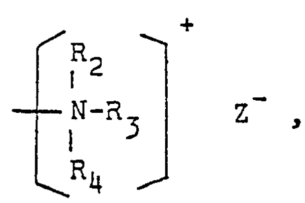 ,

(II") (III") (IV")

bezeichnete Gruppe substituiert oder durch eine gleiche oder eine andere Gruppe ausgewählt aus Hydroxy, Halogen, Thiocyanat und Phenyl in der 2-Stellung oder einer von Y weiter entfernten Stellung mono- oder poly-substituiert ist) ;

(b) Mono- oder Di-($C_1$-$C_4$)-alkylcyclohexyl ;

(c) Cyclohexenyl oder $C_3$-$C_4$-Alkenyl (das durch Halogen, Hydroxy, Phenyl, Halogenophenyl oder $C_1$-$C_4$-Alkylphenyl substituiert sein kann) ;

(d) Naphthyl oder Phenyl (das in einigen Fällen durch $C_1$-$C_4$-Alkyl, $C_1$-$C_3$-Halogenoalkyl, $CF_3$, $NO_2$, CN, SCN, CHO, $C_1$-$C_3$-Alkoxy, $C_1$-$C_3$-Alkoxycarbonyl, Aminocarbonyl, Di-$C_1$-$C_4$-alkylamino oder $C_1$-$C_2$-Alkylthio mono- oder poly-substituiert ist und weiterhin auch Halogen enthalten kann) ;

oder,

wenn Y O ist, $R_1$ auch für

(e) geradkettiges oder kettenverzweigtes $C_3$-$C_6$-Alkinyl (das in einigen Fällen durch $C_1$-$C_4$-Alkyl, $C_1$-$C_4$-Alkoxy, Halogen, Phenyl, Halogenophenyl oder $C_1$-$C_4$-Alkylphenyl mono- oder polysubstituiert ist);

(f) Halogenocyclohexyl, das gegebenenfalls durch $C_1$-$C_4$-Alkyl substituiert ist;

(g) eine durch eine der nachstehenden Formeln bezeichnete Gruppe

$-R_1'-O-R_5$ ,
(V")

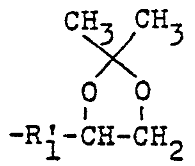 ,
(VI")

$-R_1'-O-CO-R_6$ ,
(VII")

$-R_1'-O-CO-O-R_7$ ,
(VIII")

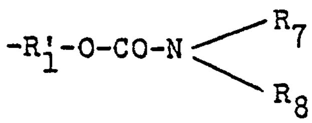 ,
(IX")

$-R_1'-CO-R_9$ ,
(X")

$-R_1'-O-SO_2-R_7$ oder
(XI")

$-R_1'-S(O)_p-R_7$ ;
(XII")

(h) $C_1$-$C_2$-Alkyl, das durch Furyl, Tetrahydrofuryl, Pyridyl oder Oxiranyl substituiert ist;

(i) $C_2$-$C_4$-Alkyl, das durch 3 bis 7 Chlor- oder Fluor-Atome substituiert ist;

oder,

wenn Y S ist, $R_1$ für

(k) $C_3$-$C_4$-Alkenyl

steht,

herbizide Wirksamkeit besitzen,

wobei in den vorstehenden Formeln

$R_2$ Wasserstoff, $C_1$-$C_4$-Alkyl, $C_1$-$C_4$-Alkoxy, Hydroxyethyl oder Chloroethyl bezeichnet und

$R_3$ Wasserstoff, $C_1$-$C_4$-Alkyl, Chloroethyl, Phenyl, Halogenophenyl, $C_1$-$C_4$-Alkylphenyl, Hydroxyethyl oder aliphatisches $C_1$-$C_4$-Acyl bezeichnet, oder

$R_2$ und $R_3$ zusammen genommen eine 2-, 4- oder 5-gliedrige, gesättigte oder ungesättigte Kohlenwasserstoff-Kette bezeichnen, von der ein Kohlenstoff-Atom durch —O—, —CO—, —N—, —NH— oder —N($C_1$-$C_4$-Alkyl)— ersetzt sein kann,

$R_4$ Wasserstoff oder $C_1$-$C_4$-Alkyl bezeichnet,

$Z^-$ für ein anorganisches oder organisches Säure-Anion steht,

$R_1'$ ein geradkettiges oder kettenverzweigtes $C_1$-$C_{12}$-Alkylen bezeichnet,

$R_5$ $C_1$-$C_6$-Alkyl, $C_1$-$C_4$-Halogenoalkyl, $C_2$-$C_8$-Alkoxyalkyl, $C_3$-$C_{12}$-Alkoxyalkoxy, Hydroxyethyl oder Phenyl, das gegebenenfalls durch Halogen und/oder $C_1$-$C_3$-Alkyl mono- oder di-substituiert ist, bezeichnet,

$R_6$ Wasserstoff, $C_1$-$C_4$-Alkyl, $C_1$-$C_4$-Halogenoalkyl, Phenyl, das gegebenenfalls durch Halogen, Nitro und/oder $C_1$-$C_4$-Alkyl substituiert ist, oder eine Gruppe der allgemeinen Formeln

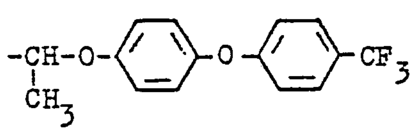

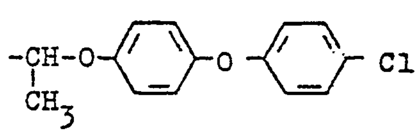

oder

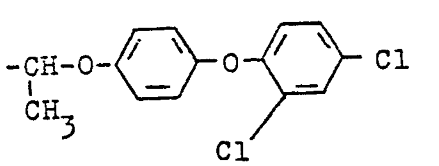

bezeichnet,

$R_7$ $C_1$-$C_4$-Alkyl, Phenyl, Halogenophenyl, Nitrophenyl oder $C_1$-$C_4$-Alkylphenyl bezeichnet,

$R_8$ Wasserstoff, $C_1$-$C_4$-Alkyl oder $C_1$-$C_4$-Alkoxy bezeichnet,
$R_9$ $C_1$-$C_4$-Alkyl bezeichnet und
p 0, 1 oder 2 ist.

Weiterhin wurde in der DE-PS 2 812 571 (entsprechend der JP-OS 54-119 476 offenbart, daß Verbindungen der allgemeinen Formel

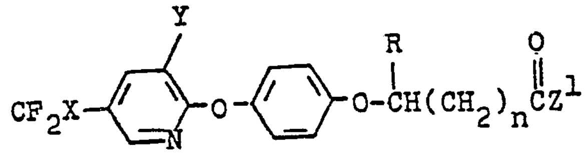

in der

X für Fluor oder Chlor,
Y für Wasserstoff oder Chlor,
R für Wasserstoff, Methyl oder Ethyl stehen,
n 0 oder 2 ist und
$Z^1$ Hydroxy, $C_1$-$C_6$-Alkoxy, dessen Alkyl-Struktureinheit gegebenenfalls durch 1 bis 3 Halogen substituiert ist, $C_1$-$C_4$-Alkoxy-$C_1$-$C_4$-alkoxy, $C_2$-$C_4$-Alkenyloxy, $C_2$-$C_4$-Alkinyloxy, $C_3$-$C_6$-Cycloalkyl, dessen Cycloalkyl-Struktureinheit gegebenenfalls durch $C_1$-$C_4$-Alkyl substituiert ist, $C_1$-$C_4$-Alkoxycarbonyl-$C_1$-$C_4$-alkoxy, Phenoxy, dessen Phenyl-Struktureinheit gegebenenfalls durch 1 bis 3 Halogen oder $C_1$-$C_4$-Alkyl substituiert ist, Benzyloxy, Glycidyloxy, $C_1$-$C_4$-Alkylthio, $C_2$-$C_4$-Alkenylthio, Phenylthio, dessen Phenyl-Struktureinheit gegebenenfalls durch 1 bis 3 Halogen oder $C_1$-$C_4$-Alkyl substituiert ist, Amino, $C_1$-$C_4$-Alkylamino, $C_1$-$C_4$-Alkoxycarbonylmethylamino, Hydroxycarbonylmethylamino, eine Anilino-Gruppe, deren Phenyl-Struktureinheit gegebenenfalls durch 1 bis 3 Halogen substituiert sein kann, Pyridin-2-ylamino, ein —O— Kation oder Halogen bezeichnet,
herbizide Wirksamkeit besitzen.

Gegenstand der vorliegenden Erfindung sind nunmehr, als neue Verbindungen, substituierte Phenoxypropionate der allgemeinen Formel

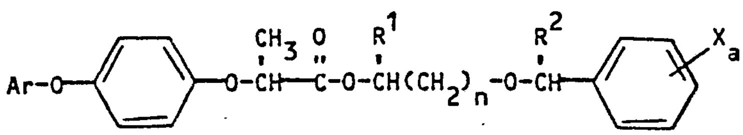

(I)

in der

$R^1$ und $R^2$ jeweils unabhängig voneinander ein Wasserstoff-Atom oder eine $C_1$- bis $C_6$-Alkyl-Gruppe bezeichnen,
X für ein Wasserstoff- oder Halogen-Atom oder eine Nitro-, $C_1$- bis $C_6$-Alkyl- oder $C_1$- bis $C_6$-Alkoxy-Gruppe steht,
a und n jeweils unabhängig voneinander 1 oder 2 sind und
Ar eine Gruppe der allgemeinen Formeln

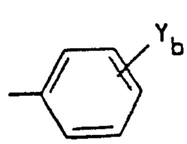

oder

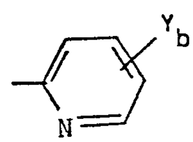

(Ia) (Ib)

bezeichnet, in der

Y für eine Trifluoromethyl-Gruppe, ein Halogen-Atom oder eine Nitro-, Cyano oder $C_1$ bis $C_6$-Alkyl-Gruppe steht und
b 1, 2 oder 3 ist.

Gegenstand der vorliegenden Erfindung ist weiter ein Verfahren zur Herstellung einer Verbindung der vorliegenden Erfindung, das dadurch gekennzeichnet ist, daß

(a) eine Verbindung der allgemeinen Formel

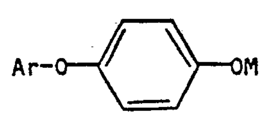

(II)

in der

Ar die im Vorstehenden angegebene Bedeutung hat und

M ein Wasserstoff-Atom oder ein Alkalimetall-Atom bezeichnet, mit einer Verbindung der allgemeinen Formel

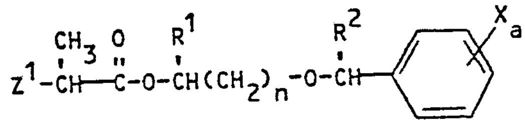

(III)

in der

$R^1$, $R^2$, X, a und n die im Vorstehenden angegebenen Bedeutungen haben und

$Z^1$ für ein Halogen-Atom steht,

umgesetzt wird,

(b) eine Verbindung der allgemeinen Formel

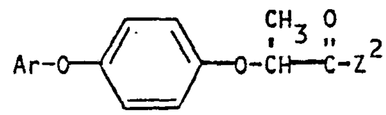

(IV)

in der

Ar die im Vorstehenden angegebene Bedeutung hat und

$Z^2$ für eine Hydroxyl-Gruppe oder ein Halogen-Atom steht,

mit einer Verbindung der allgemeinen Formel

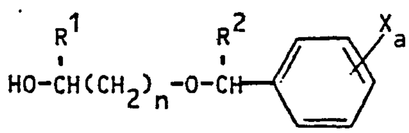

(V)

in der $R^1$, $R^2$, X, a und n die im Vorstehenden angegebenen Bedeutungen haben, umgesetzt wird oder

(c) eine Verbindung der allgemeinen Formel

$$AR—Z^1, \quad (VI),$$

in der Ar und $Z^1$ die im Vorstehenden angegebenen Bedeutungen haben, mit einer Verbindung der allgemeinen Formel

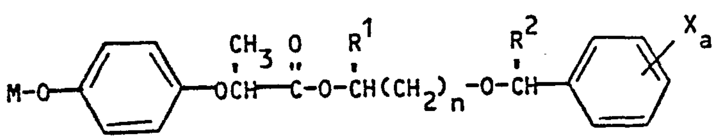

(VII)

in der $R^1$, $R^2$, X, M a und n die im Vorstehenden angegebenen Bedeutungen haben, umgesetzt wird.

Überraschenderweise können die substituierten Phenoxypropionat-Verbindungen gemäß der vorliegenden Erfindung, die bisher in der Literatur nicht beschrieben wurden, leicht in hohen Ausbeuten synthetisiert werden und sind neue aktive Verbindungen, die eine ausgezeichnete selektive herbizide Wirksamkeit gegenüber grasartigen Unkräutern aufweisen, ohne eine nennenswerte Phytotoxizität gegenüber landwirtschaftlichen Nutzpflanzen zu entfalten. Es ist insbesondere überraschend, daß die Verbindungen gemäß der vorliegenden Erfindung hervorragende Eigenschaften besitzen, die in bezug auf ihre Struktur ähnliche Verbindungen gemäß dem Stand der Technik nicht aufweisen, vor allem insofern, als sie bereits in kleinen Mengen eine ausreichende herbizide Wirkung zeigen und dabei für Nutzpflanzen gut verträglich sind und außerdem auch der Regenerierung von Unkräutern, insbesondere von perennierenden grasartigen Unkräutern, infolge ihrer ausgezeichneten Langzeitwirkung über eine lange Zeitdauer hinweg erfolgreich entgegenwirken.

Bevorzugte Verbindungen gemäß der vorliegenden Erfindung und die ihnen entsprechenden Ausgangsstoffe sind diejenigen, in denen

Ar eine Gruppe der Formel (Ia) oder (Ib) bezeichnet, in der

Y für eine Trifluoromethyl-Gruppe, ein Fluor-, Chlor-, Brom- oder Iod-Atom, oder eine Nitro-, Cyano-, Methyl-, Ethyl-, Propyl-, Isopropyl-, n-, iso-, sec- oder tert-Butyl-Gruppe steht;

b 1, 2 oder 3 ist;

$R^1$ und $R^2$ jeweils unabhängig voneinander ein Wasserstoff-Atom oder eine Methyl-, Ethyl-, n-Propyl-, Isopropyl, n-, iso-, sec- oder tert-Butyl-Gruppe bezeichnen;

X für ein Wasserstoff-, Fluor-, Chlor-, Brom-, oder Iod-Atom, eine Nitro-, Methyl-, Methoxy-, Ethyl-, Ethoxy-, n-Propyl-, n-Propoxy-, Isopropyl-, Isopropoxy- oder n-, iso-, sec- oder tert-Butyl oder -Butoxy-Gruppe steht und

a und n jeweils unabhängig voneinander 1 oder 2 sind.

Wenn 4-(4-Trifluoromethylphenoxy) phenol und 2-Benzyloxyethyl-2-bromo-propionat als Ausgangsstoffe eingesetzt werden, wird der Ablauf der Reaktionsvariante (a) gemäß der vorliegenden Erfindung durch die folgende Gleichung beschrieben :

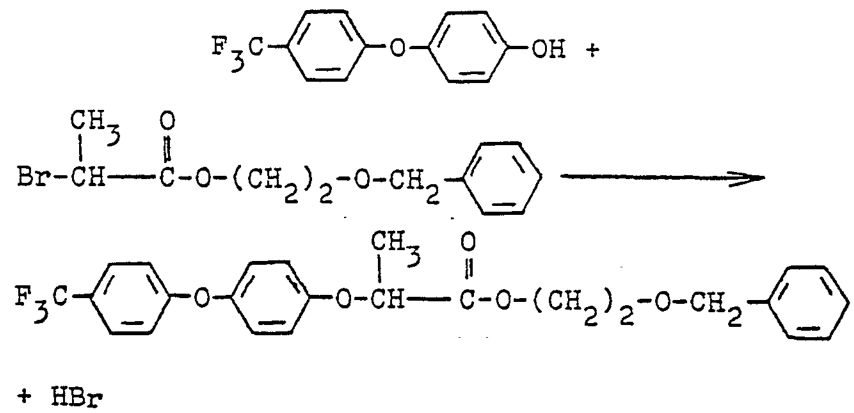


Wenn 2-[4-(3,5-Dichloro-2-pyridyloxy) phenoxy] propionylchlorid und 1-Benzyloxy-2-propanol als Ausgangsstoffe eingesetzt werden, wird der Ablauf der Reaktionsvariante (b) gemäß der vorliegenden Erfindung durch die folgende Gleichung beschrieben :

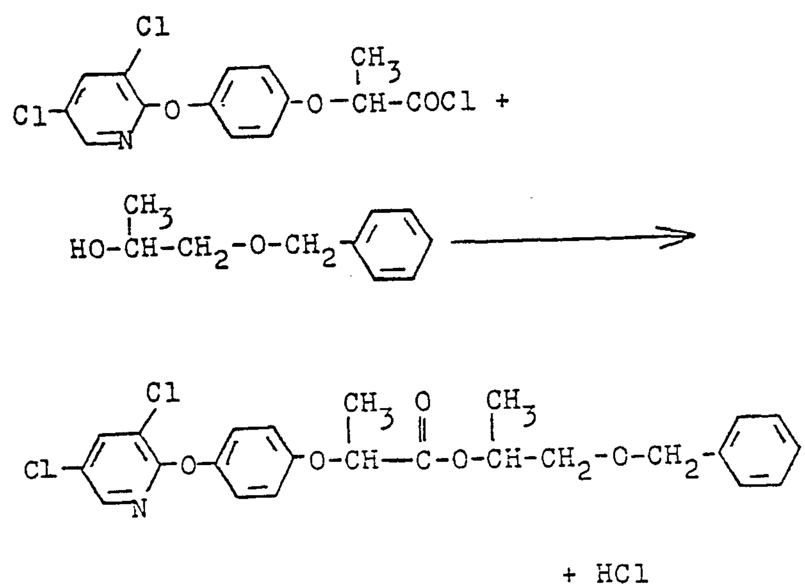


Wenn 4-Trifluoromethylphenylchlorid und 2-(2-Fluorobenzyloxy) ethyl-2-(4-hydroxyphenoxy) propionat als Ausgangsstoffe eingesetzt werden, wird der Ablauf der Reaktionsvariante (c) gemäß der vorliegenden Erfindung durch die folgende Gleichung beschrieben :

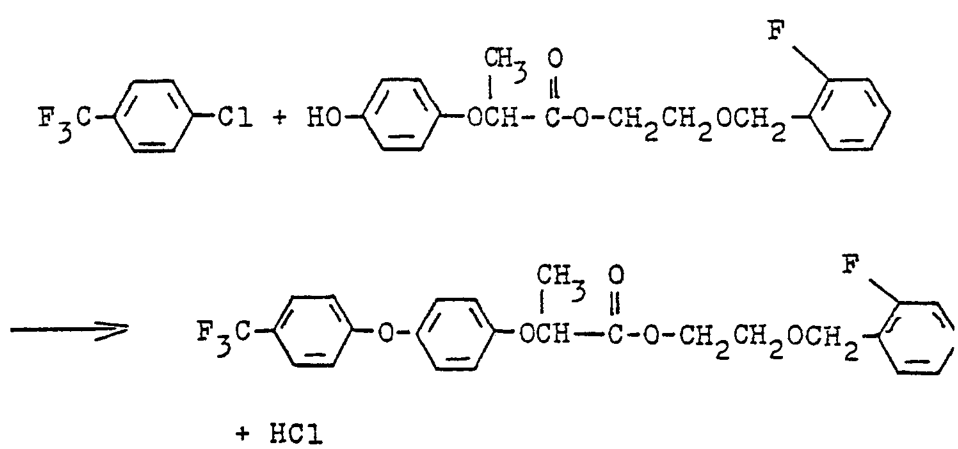

Beispiele für die als Ausgangsstoffe bei der Reaktionsvariante (a) gemäß der vorliegenden Erfindung eingesetzten Verbindungen der Formel (II) sind :

4-(4-Trifluoromethylphenoxy) phenol,
4-(2-Trifluoromethylphenoxy) phenol,
4-(4-Fluorophenoxy) phenol,
4-(2,4-Dichlorophenoxy) phenol,
4-(2-Chloro-4-nitrophenoxy) phenol,
4-(2-Trifluoromethyl-4-chlorophenoxy) phenol,
4-(4-Trifluoromethyl-2-chlorophenoxy) phenol,
4-(3,5-Dichloro-2-pyridyloxy) phenol,
4-(5-Nitro-2-pyridyloxy) phenol,
4-(4-Nitrophenoxy) phenol,
4-(4-Bromo-2-chlorophenoxy) phenol,
4-(4-Trifluoromethyl-2-nitrophenoxy) phenol,
4-(2,6-Dichloro-4-trifluoromethyl-phenoxy) phenol,
4-(2-Cyano-4-trifluoromethylphenoxy) phenol,
4-(2-Chloro-4-cyanophenoxy) phenol,
4-(3-Chloro-5-nitro-2-pyridyloxy) phenol,
4-(5-Trifluoromethyl-2-pyridyloxy) phenol,
4-(3-Chloro-5-trifluoromethyl-2-pyridyloxy) phenol,
4-(5-Bromo-3-chloro-2-pyridyloxy) phenol und
4-(4-Chloro-2-methylphenoxy) phenol sowie deren Alkalimetall- (z. B. Li-, Na- und K-) -Salze.

Beispiele für die anderen Ausgangsstoffe der Formel (III) zum Einsatz bei der Reaktionsvariante (a) sind :

2-Benzyloxyethyl-2-chloro- (oder -bromo-) propionat,
3-Benzyloxypropyl-2-chloro- (oder -bromo-) propionat,
1-Methyl-2-benzyloxyethyl-2-chloro- (oder -bromo-)-propionat,
2-α-Methylbenzyloxyethyl-2-chloro- (oder -bromo-)-propionat,
2-(2-Fluorobenzyloxy) ethyl-2-chloro- (oder -bromo-)-propionat,
2-(4-Fluorobenzyloxy) ethyl-2-chloro- (oder -bromo-)-propionat,
2-(2-Chlorobenzyloxy) ethyl-2-chloro- (oder -bromo-)-propionat,
2-(4-Chlorobenzyloxy) ethyl-2-chloro- (oder -bromo-)-propionat,
2-(2,4-Dichlorobenzyloxy) ethyl-2-chloro- (oder -bromo-) propionat,
2-(3,4-Dichlorobenzyloxy) ethyl-2-chloro- (oder -bromo-) propionat,
2-(2,6-Dichlorobenzyloxy) ethyl-2-chloro- (oder -bromo-) propionat,
2-(2-Methylbenzyloxy) ethyl-2-chloro- (oder -bromo-)-propionat,
2-(3-Nitrobenzyloxy) ethyl-2-chloro- (oder -bromo-)-propionat,
2-(4-Methoxybenzyloxy) ethyl-2-chloro- (oder -bromo-)-propionat,
2-(4-Bromobenzyloxy) ethyl-2-chloro- (oder -bromo-)-propionat,
1-Methyl-2-α-methylbenzyloxyethyl-2-chloro- (oder -bromo-) propionat,
2-(3-Chlorobenzyloxy) ethyl-2-chloro- (oder -bromo-)-propionat,
2-(2-Bromobenzyloxy) ethyl-2-chloro- (oder -bromo-)-propionat,
2-(3-Fluorobenzyloxy) ethyl-2-chloro- (oder -bromo-)-propionat,
1-Methyl-2-(2-fluorobenzyloxy) ethyl-2-chloro- (oder -bromo-) propionat,
3-(2-Fluorobenzyloxy) propyl-2-chloro- (oder -bromo-)-propionat,
2-(2-Nitrobenzyloxy) ethyl-2-chloro- (oder -bromo-)-propionat,
2-(4-Nitrobenzyloxy) ethyl-2-chloro- (oder -bromo-)-propionat,
2-(4-Methylbenzyloxy) ethyl-2-chloro- (oder -bromo-)-propionat,
2-(2-Methoxybenzyloxy) ethyl-2-chloro- (oder -bromo-)-propionat und
3-(4-Fluorobenzyloxy) propyl-2-chloro- (oder -bromo-)-propionat.

Die Reaktionsvariante (a) gemäß der vorliegenden Erfindung wird vorzugsweise in Gegenwart eines Verdünnungsmittels durchgeführt. Zu diesem Zweck können sämtliche inerten Lösungsmittel verwendet werden.

Beispiele für solche Lösungsmittel oder Verdünnungsmittel sind Wasser ; aliphatische, alicyclische und aromatische Kohlenwasserstoffe, die jeweils gegebenenfalls chloriert sein können, (wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol) ; Ether (wie Diethylether, Methylethylether, Diisopropylether, Dibutylether, Propylenoxid, Dioxan und Tetrahydrofuran) ; Ketone (wie Aceton, Methylethylketon, Methylisopropylketon und Methylisobutylketon) ; Nitrile (wie Acetonitril, Propionitril und Acrylnitril) ; Alkohole (wie Methanol, Ethanol, Isopropanol, Butanol und Ethylenglycol) ; Ester (wie Ethylacetat und Amylacetat) ; Säureamide (wie Dimethylformamid und Dimethylacetamid) ; Sulfone und Sulfoxide (wie Dimethylsulfoxid und Sulfolan) und Basen (wie Pyridin).

Die Reaktionsvariante (a) wird vorzugsweise in Gegenwart eines säurebindenden Mittels durchgeführt. Als Beispiele für solche säurebindenden Mittel seien Hydroxide, Carbonate, Hydrogencarbonate und Alkoholate von Alkalimetallen und tertiäre Amine wie Triethylamin, Diethylanilin und Pyridin erwähnt.

Die Reaktionsvariante (a) kann innerhalb eines breiten Temperaturbereichs durchgeführt werden. Im allgemeinen wird sie bei einer Temperatur zwischen − 20 °C und dem Siedepunkt der Reaktionsmischung, vorzugsweise zwischen 0 °C und 100 °C, durchgeführt.

Diese Reaktionsvariante wird vorzugsweise unter dem Druck der Umgebung durchgeführt, wenngleich sie auch bei erhöhtem oder vermindertem Druck durchgeführt werden kann.

Beispiele für die als Ausgangsstoffe bei der Reaktionsvariante (b) eingesetzten Verbindungen der Formel (IV) sind :

2-[4-(4-Trifluoromethylphenoxy) phenoxy] propionylchlorid,
2-[4-(2-Trifluoromethylphenoxy) phenoxy] propionylchlorid,
2-[4-(4-Fluorophenoxy) phenoxy] propionylchlorid,
2-[4-(2,4-Dichlorophenoxy) phenoxy] propionylchlorid,
2-[4-(2-Chloro-4-nitrophenoxy) phenoxy] propionylchlorid,
2-[4-(2-Trifluoromethyl-4-chlorophenoxy) phenoxy]-propionylchlorid,
2-[4-(3,5-Dichloro-2-pyridyloxy) phenoxy] propionyl-chlorid,
2-[4-(4-Trifluoromethyl-2-chlorophenoxy) phenoxy]-propionylchlorid,
2-[4-(5-Nitro-2-pyridyloxy) phenoxy] propionylchlorid,
2-[4-(4-Nitrophenoxy) phenoxy] propionylchlorid,
2-[4-(4-Bromo-2-chlorophenoxy) phenoxy] propionylchlorid,
2-[4-(4-Trifluoromethyl-2-nitrophenoxy) phenoxy]-propionylchlorid,
2-[4-(2,6-Dichloro-4-trifluoromethylphenoxy) phenoxy] propionylchlorid,
2-[4-(2-Cyano-4-trifluoromethylphenoxy) phenoxy]-propionylchlorid,
2-[4-(2-Chloro-4-cyanophenoxy) phenoxy] propionylchlorid,
2-[4-(3-Chloro-5-nitro-2-pyridyloxy) phenoxy] propionylchlorid,
2-[4-(5-Trifluoromethyl-2-pyridyloxy) phenoxy] propionylchlorid,
2-[4-(3-Chloro-5-trifluoromethyl-2-pyridyloxy) phenoxy] propionylchlorid,
2-[4-(5-Bromo-3-chloro-2-pyridyloxy) phenoxy] propionylchlorid und
2-[4-(4-Chloro-2-methylphenoxy) phenoxy] propionylchlorid,

sowie die entsprechenden Bromide oder Derivate mit freien Propionsäure-Gruppen.

Beispiele für die anderen Ausgangsstoffe der Formel (V) zum Einsatz bei der Reaktionsvariante (b) sind :

2-Benzyloxyethanol,
3-Benzyloxypropanol,
1-Benzyloxy-2-propanol,
2-α-Methylbenzyloxyethanol,
2-(2-Fluorobenzyloxy) ethanol,
2-(4-Fluorobenzyloxy) ethanol,
2-(2-Chlorobenzyloxy) ethanol,
2-(4-Chlorobenzyloxy) ethanol,
2-(2,4-Dichlorobenzyloxy) ethanol,
2-(3,4-Dichlorobenzyloxy) ethanol,
2-(2,6-Dichlorobenzyloxy) ethanol,
2-(2-Methylbenzyloxy) ethanol,
2-(3-Nitrobenzyloxy) ethanol,
2-(4-Methoxybenzyloxy) ethanol,
2-(4-Bromobenzyloxy) ethanol,
1-(α-Methylbenzyloxy)-2-propanol,
2-(3-Chlorobenzyloxy) ethanol,
2-(2-Bromobenzyloxy) ethanol,
2-(3-Fluorobenzyloxy) ethanol,
1-(2-Fluorobenzyloxy)-2-propanol,
3-(2-Fluorobenzyloxy) propanol,
2-(2-Nitrobenzyloxy) ethanol,
2-(4-Nitrobenzyloxy) ethanol,
2-(4-Methylbenzyloxy) ethanol,
2-(2-Methoxybenzyloxy) ethanol und
3-(4-Fluorobenzyloxy) propanol.

Bei der Durchführung der Reaktionsvariante (b) wird vorzugsweise eines der im Vorstehenden für die Reaktionsvariante (a) beschriebenen Lösungsmittel oder Verdünnungsmittel verwendet, um das Endprodukt mit hoher Reinheit in hoher Ausbeute zu erhalten. Die Reaktionsvariante (b) wird gleichermaßen vorzugsweise in Gegenwart eines säurebindenden Mittels, wie für die Reaktionsvariante (a) beschrieben, durchgeführt.

Die Reaktionsvariante (b) kann unter den gleichen Reaktionsbedingungen in bezug auf Temperatur und Druck durchgeführt werden, wie sie für die Reaktionsvariante (a) im Vorstehenden genannt wurden.

Beispiele für die als Ausgangsstoffe bei der Reaktionsvariante (c) eingesetzten Verbindungen der Formel (VI) sind :

4-(oder 2-) Trifluoromethylphenylchlorid,
4-Fluorophenylchlorid,
2,4-Dichlorophenylchlorid,
2-Chloro-4-nitrophenylchlorid,
4-Chloro-2-trifluoromethylphenylchlorid,
2-Chloro-4-trifluoromethylphenylchlorid,
3,5-Dichloro-2-pyridinylchlorid,
5-Nitropyridinylchlorid,
4-Nitrophenylchlorid,
4-Bromo-2-chlorophenylchlorid,
2-Nitro-4-trifluoromethylphenylchlorid,
2,6-Dichloro-4-trifluoromethylphenylchlorid,
2-Cyano-4-trifluoromethylphenylchlorid,
2-Chloro-4-cyanophenylchlorid,
3-Chloro-5-nitro-2-pyridinylchlorid,
5-Trifluoromethyl-2-pyridinylchlorid,
3-Chloro-5-trifluoromethyl-2-pyridinylchlorid,
5-Bromo-3-chloro-2-pyridinylchlorid und
4-Chloro-2-methylphenylchlorid,
sowie die entsprechenden Bromide.

Beispiele für die anderen Ausgangsstoffe der Formel (VII), die ebenfalls bei der Reaktionsvariante (c) eingesetzt werden, sind :

2-Benzyloxyethyl-2-(4-hydroxyphenoxy) propionat,
3-Benzyloxypropyl-2-(4-hydroxyphenoxy) propionat,
1-Methyl-2-benzyloxyethyl-2-(4-hydroxyphenoxy) propionat,
2-α-Methylbenzyloxyethyl-2-(4-hydroxyphenoxy) propionat,
2-(2-Fluorobenzyloxy) ethyl-2-(4-hydroxyphenoxy) propionat,
2-(4-Fluorobenzyloxy) ethyl-2-(4-hydroxyphenoxy) propionat,
2-(2-Chlorobenzyloxy) ethyl-2-(4-hydroxyphenoxy) propionat,
2-(4-Chlorobenzyloxy) ethyl-2-(4-hydroxyphenoxy) propionat,
2-(2,4-Dichlorobenzyloxy) ethyl-2-(4-hydroxyphenoxy)-propionat,
2-(3,4-Dichlorobenzyloxy) ethyl-2-(4-hydroxyphenoxy)-propionat,
2-(2,6-Dichlorobenzyloxy) ethyl-2-(4-hydroxyphenoxy)-propionat,
2-(2-Methylbenzyloxy) ethyl-2-(4-hydroxyphenoxy) propionat,
2-(3-Nitrobenzyloxy) ethyl-2-(4-hydroxyphenoxy) propionat,
2-(4-Methoxybenzyloxy) ethyl-2-(4-hydroxyphenoxy) propionat,
2-(4-Bromobenzyloxy) ethyl-2-(4-hydroxyphenoxy) propionat,
1-Methyl-2-α-methylbenzyloxyethyl-2-(4-hydroxyphenoxy) propionat,
2-(3-Chlorobenzyloxy) ethyl-2-(4-hydroxyphenoxy) propionat,
2-(2-Bromobenzyloxy) ethyl-2-(4-hydroxyphenoxy) propionat,
2-(3-Fluorobenzyloxy) ethyl-2-(4-hydroxyphenoxy) propionat,
1-Methyl-2-(2-fluorobenzyloxy) ethyl-2-(4-hydroxyphenoxy) propionat,
3-(2-Fluorobenzyloxy) propyl-2-(4-hydroxyphenoxy) propionat,
2-(2-Nitrobenzyloxy) ethyl-2-(4-hydroxyphenoxy) propionat,
2-(4-Nitrobenzyloxy) ethyl-2-(4-hydroxyphenoxy) propionat,
2-(2-Methoxybenzyloxy) ethyl-2-(4-hydroxyphenoxy) propionat,
3-(4-Fluorobenzyloxy) propyl-2-(4-hydroxyphenoxy) propionat und
2-(4-Methylbenzyloxy) ethyl-2-(4-hydroxyphenoxy) propionat.

Bei der Durchführung der Reaktionsvariante (c) wird vorzugsweise eines der im Vorstehenden für die Reaktionsvariante (a) beschriebenen Lösungsmittel oder Verdünnungsmittel verwendet, um das Endprodukt mit hoher Reinheit in hoher Ausbeute zu erhalten. Die Reaktionsvariante (c) wird gleichermaßen vorzugsweise in Gegenwart eines säurebindenden Mittels, wie für die Reaktionsvariante (a) beschrieben, durchgeführt.

Die Reaktionsvariante (c) kann unter den gleichen Reaktionsbedingungen in bezug auf Temperatur

und Druck durchgeführt werden, wie sie für die Reaktionsvariante (a) im Vorstehenden genannt wurden.

Die als Zwischenprodukte der Formel (III) auftretenden Verbindungen (die auch als Zwischenprodukte für die Herstellung der Zwischenprodukte der Formel (VII) verwendet werden können) sind bisher in der Literatur nicht offenbart worden und bilden einen weiteren Gegenstand der vorliegenden Erfindung.

Die vorliegende Erfindung macht weiterhin ein Verfahren zur Herstellung einer Verbindung der allgemeinen Formel (III) verfügbar, das dadurch gekennzeichnet ist, daß eine Verbindung der allgemeinen Formel

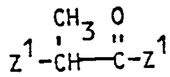

(VIII)

in der jedes $Z^1$ unabhängig für ein Halogen-Atom steht,
mit einer Verbindung der allgemeinen Formel

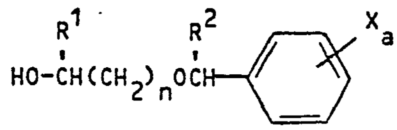

(IX)

in der $R^1$, $R^2$, X, a und n die im Vorstehenden angegebenen Bedeutungen haben, umgesetzt wird.

Beispiele für die Verbindungen der Formel (VIII), die als Ausgangsstoffe bei der Herstellung von Verbindungen der Formel (III) eingesetzt werden, sind

2-Bromopropionylbromid und
2-Chloropropionylchlorid.

Beispiele für die anderen Ausgangsstoffe der Formel (IX), die ebenfalls bei der Herstellung von Verbindungen der Formel (III) eingesetzt werden, sind :

2-Benzyloxyethanol,
3-Benzyloxypropanol,
1-Methyl-2-benzyloxyethanol,
2-α-Methylbenzyloxyethanol,
2-(2-Chlorobenzyloxy) ethanol,
2-(3-Chlorobenzyloxy) ethanol,
2-(4-Chlorobenzyloxy) ethanol,
2-(2,4-Dichlorobenzyloxy) ethanol,
2-(2,6-Dichlorobenzyloxy) ethanol,
2-(3,4-Dichlorobenzyloxy) ethanol,
2-(2-Methylbenzyloxy) ethanol,
2-(4-Methylbenzyloxy) ethanol,
2-(2-Methoxybenzyloxy) ethanol,
2-(4-Methoxybenzyloxy) ethanol,
2-(2-Bromobenzyloxy) ethanol,
2-(4-Bromobenzyloxy) ethanol,
1-Methyl-2-α-methylbenzyloxyethanol,
2-(2-Fluorobenzyloxy) ethanol,
2-(3-Fluorobenzyloxy) ethanol,
2-(4-Fluorobenzyloxy) ethanol,
2-(2-Nitrobenzyloxy) ethanol,
2-(3-Nitrobenzyloxy) ethanol,
2-(4-Nitrobenzyloxy) ethanol,
1-Methyl-2-(2-fluorobenzyloxy) ethanol,
3-(2-Fluorobenzyloxy) propanol,
3-(4-Fluorobenzyloxy) propanol.

Wenn 2-Bromopropionylbromid und 2-Benzyloxyethanol als Ausgangsstoffe eingesetzt werden, wird der Reaktionsablauf durch die folgende Gleichung beschrieben :

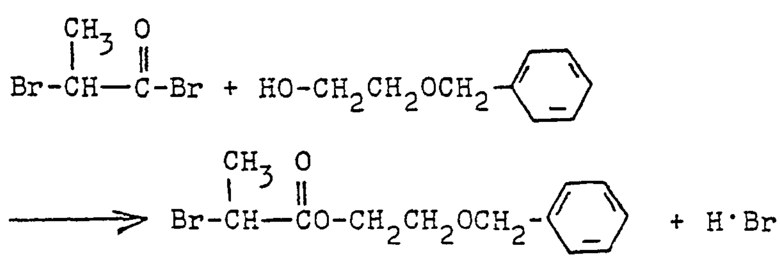

Bei dem Verfahren zur Herstelung von Verbindungen der Formel (III) wird vorzugsweise eines der im Vorstehenden für die Reaktionsvariante (a) beschriebenen Lösungsmittel oder Verdünnungsmittel verwendet, um die Endprodukte mit hoher Reinheit in hoher Ausbeute zu erhalten. Diese Reaktion wird gleichermaßen vorzugsweise in Gegenwart eines säurebindenden Mittels, wie für die Reaktionsvariante (a) beschrieben, durchgeführt.

Die Reaktionsbedingungen in bezug auf Temperatur und Druck werden ebenfalls aus denjenigen gewählt, die für die Reaktionsvariante (a) im Vorstehenden genannt wurden.

Auch die Ausgangs-Verbindungen der Formel (VII) sind neu und bilden einen weiteren Gegenstand der vorliegenden Erfindung.

Die vorliegende Erfindung macht weiterhin ein Verfahren zur Herstellung einer Verbindung der allgemeinen Formel (VII) verfügbar, das dadurch gekennzeichnet ist, daß eine Verbindung der Formel

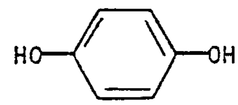

mit einer Verbindung der allgemeinen Formel

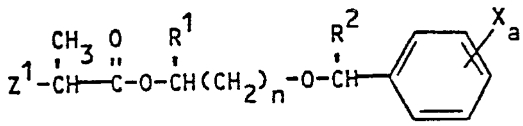

(III)

in der $R^1$, $R^2$, X, a, n und $Z^1$ die im Vorstehenden angegebenen Bedeutungen haben, umgesetzt wird.

Beispiele für die Verbindungen der Formel (III), die als Ausgangsstoffe eingesetzt werden, wurden bereits als bevorzugte Ausgangsstoffe für die Reaktionsvariante (a) genannt und können wie im Vorstehenden beschrieben hergestellt werden.

Wenn Hydrochinon und 2-Benzyloxyethyl-2-bromopropionat als Ausgangsstoffe eingesetzt werden, wird der Ablauf der Reaktion zur Herstellung der Verbindungen der Formel (VII) durch die folgende Gleichung beschrieben :

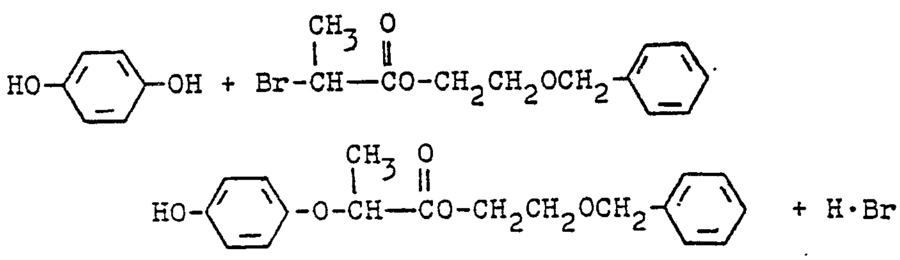

Bei dem Verfahren zur Herstellung von Verbindungen der Formel (VII) wird vorzugsweise eines der im Vorstehenden für die Reaktionsvariante (a) beschriebenen inerten Lösungsmittel oder Verdünnungsmittel verwendet, um die Endprodukte mit hoher Reinheit in hoher Ausbeute zu erhalten. Diese Reaktion wird gleichermaßen vorzugsweise in Gegenwart eines säurebindenden Mittels, wie für die Reaktionsvariante (a) beschrieben, durchgeführt.

Die Reaktionsbedingungen in bezug auf Temperatur und Druck werden ebenfalls aus denjenigen gewählt, die für die Reaktionsvariante (a) im Vorstehenden genannt wurden.

Die wirksamen Verbindungen der Formel (I) gemäß der vorliegenden Erfindung zeigen eine ausgezeichnete selektive herbizide Wirkung, wenn sie als Mittel zur Bodenbehandlung vor oder nach dem Auflaufen der grasartigen Unkräuter eingesetzt werden.

Da die aktiven Verbindungen gemäß der vorliegenden Erfindung nur geringe oder gar keine Toxizität gegenüber warmblütigen Tieren zeigen sowie eine gute Selektivität gegenüber landwirtschaftlichen Nutzpflanzen aufweisen, das heißt keine Phytotoxizität gegenüber landwirtschaftlichen Nutzpflanzen zeigen, können sie vorteilhaft als Herbizide zur Unkrautbekämpfung eingesetzt werden.

Die Wirkstoffe gemäß der vorliegenden Erfindung beeinflussen das Wachstum der Pflanzen und können dementsprechend als Entlaubungsmittel, Abbrandmittel, Mittel zur Zerstörung breitblättriger Pflanzen, keimungshemmende Mittel und, insbesondere, als Unkrautvernichtungsmittel verwendet werden. Unter « Unkräutern » im weitesten Sinne sind solche Pflanzen zu verstehen, die an Stellen wachsen, wo sie nicht erwünscht sind.

Ob die erfindungsgemäßen Verbindungen totale oder selektive Herbizide-Wirkung entfalten, hängt im wesentlichen von der angewandten Menge ab.

Die aktiven Verbindungen gemäß der vorliegenden Erfindung können beispielsweise zur Bekämpfung der folgenden Pflanzen verwendet werden :

Dikotyledonen-Unkräuter der Gattungen

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Rorippa, Rotala, Lindernia, Lamium, Veronica, Abutilon, Emex, Datura, Viola, Galeopsis, Papaver, Centaurea und Solanum ; und

Monokotyledonen-Unkräuter der Gattungen

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus und Apera.

Die aktiven Verbindungen gemäß der vorliegenden Erfindung können beispielsweise als selektive Herbizide in den folgenden Kulturen verwendet werden :

Dikotyledonen-Kulturen der Gattungen

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis und Cucurbita ; und

Monokotyledonen-Kulturen der Gattungen

Oryza, Zea, Triticum, Hordeum, Avena, Secale, Sorghum, Panicum, Saccharum, Ananas, Asparagus und Allium.

Die Anwendung der erfindungsgemäßen aktiven Verbindungen ist jedoch keineswegs auf diese Gattungen begrenzt, sondern umfaßt auch andere Pflanzen in gleicher Weise.

In Abhängigkeit von den Konzentrationen können die Verbindungen zur totalen Bekämpfung von Unkräutern, beispielsweise auf Industriegelände und Bahnkörpern sowie auf Wegen und Plätzen mit oder ohne Baumbestand, verwendet werden. Ebenso können die Verbindungen auch zur Unkrautbekämpfung in perennierenden Kulturen, beispielsweise Aufforstungen, Zierbaumpflanzungen, Obstgärten, Weinbergen und -gärten, Zitrushainen, Nußbaumpflanzungen, Bananenplantagen, Kaffeeplantagen, Teeplantagen, Gummiplantagen, Ölpalmenplantagen, Kakaoplantagen Weichfruchtplantagen und Hopfenfeldern, sowie zur selektiven Unkrautbekämpfung in Jahreskulturen verwendet werden.

Die aktiven Verbindungen gemäß der vorliegenden Erfindung können insbesondere zur Bekämpfung der folgenden Pflanzen verwendet werden :

Echinochloa crus-galli,
Digitaria adscendens,
Eleusine indica,
Setaria viridis,
Avena fatua,
Alopecurus aequalis,
Setaria lutescens,
Agropyron repens oder
Agropyron tsukushiense.

Außerdem zeigen sie hevorragende herbizide sowie auch das Nachwachsen bekämpfende Wirkung gegen, beispielsweise,

Sorghum halepense oder
Cynodon dactylon.

Die Wirkstoffe können in übliche Präparate wie Lösungen, Emulsionen, benetzbare Pulver, Suspensionen, Stäubemittel, lösliche Pulver, Granulat, Suspensions-Emulsions-Konzentrate, mit dem Wirkstoff imprägnierte natürliche und synthetische Materialien und sehr feine Kapseln in polymeren Substanzen, umgewandelt werden.

Diese Präparate können in bekannter Weise hergestellt werden, beispielsweise durch Vermischen der Wirkstoffe mit Streckmitteln, d. h. flüssigen oder festen Verdünnungsmitteln oder Trägern, gegebenenfalls unter Verwendung von oberflächenaktiven Mitteln, d. h. Emulgatoren und/oder Dispergiermitteln und/oder schaumbildenden Mitteln. Bei Verwendung von Wasser als Streckmittel können beispielsweise auch organische Lösungsmittel als Hilfslösungsmittel verwendet werden.

Als flüssige Verdünnungsmittel oder Träger, insbesondere als Lösungsmittel, eignen sich hauptsächlich aromatische Kohlenwasserstoffe wie Xylol, Toluol oder Alkylnaphthaline, chlorierte aromatische oder chlorierte aliphatische Kohlenwasserstoffe wie Chlorbenzole, Chloroethylene oder Methylenchlorid, aliphatische oder alicyclische Kohlenwasserstoffe wie Cyclohexan oder Paraffine, beispielsweise Mineralöl-Fraktionen, Alkohole wie Butanol oder Glycol sowie deren Ether und Ester, Ketone wie Aceton, Methylethylketon, Methylisobutylketon oder Cyclohexanon, oder stark polare Lösungsmittel wie Dimethylformamid oder Dimethylsulfoxid, sowie auch Wasser.

Als feste Träger können gemahlene natürliche Minerale wie Kaoline, Tone, Talkum, Kreide, Quarz, Attapulgit, Montmorillonit oder Diatomeenerde, und gemahlene synthetische Minerale wie hochdisperse Kieselsäure, Aluminiumoxid und Silikate verwendet werden. Als feste Träger für Granulat können zerkleinertes und fraktioniertes natürliches Gesteinsmaterial wie Calcit, Marmor, Bimsstein, Sepiolith und Dolomit sowie synthetisches Granulat aus anorganischen oder organischen Mehlen und Granulat aus organischem Material wie Sägemehl, Kokosnußschalen, Maiskolben und Tabakstengeln verwendet werden.

Als Emulgatoren und/oder Schaumbildner können nichtionische oder anionische Emulgatoren wie Polyoxyethylen-Fettsäureester, Polyoxyethylen-Fettalkoholether, beispielsweise Alkylarylpolyglycolether,

Alkylsulfonate, Alkylsulfate, und Arylsulfonate sowie Hydrolyse-produkte des Albumins verwendet werden. Zu den Dispergiermitteln zählen beispielsweise Ligninsulfit-Ablaugen und Methylcellulose.

Haftmittel wie Carboxymethylcellulose und natürliche und synthetische Polymerisate in Form von Pulver, Granulat oder Latices, z. B. Gummiarabicum, Polyvinylalkohol und Polyvinylacetat, können in den Formulierungen verwendet werden.

Es ist möglich, farbgebende Stoffe wie anorganische Pigmente, beispielsweise Eisenoxid, Titanoxid und Preußischblau, und organische Farbstoffe wie Alizarin-Farbstoffe, Azo-Farbstoffe und Metallphthalocyanin-Farbstoffe, und Spurennährstoffe wie Salze von Eisen, Mangan, Bor, Kupfer, Cobalt, Molybdän und Zink, zu verwenden.

Die Präparate enthalten im allgemeinen von 0,001 bis 100 Gewichts-%, vorzugsweise von 0,005 bis 95 Gewichts-% des Wirkstoffes.

Die Wirkstoffe gemäß der vorliegenden Erfindung können als solche oder in Form ihrer Präparate zur Unkrautbekämpfung auch im Gemisch mit bekannten Herbiziden verwendet werden, wobei gebrauchsfertige Formulierungen oder im Tankmischverfahren erhaltene Mittel einsetzbar sind. Auch Gemische mit anderen bekannten aktiven Verbindungen wie Fungiziden, Insektiziden, Akariziden, Nematiziden, vogelabweisenden Mitteln, Wachstumsfaktoren, Pflanzennährstoffen sowie Mitteln zur Verbesserung der Bodenstruktur sind einsetzbar.

Die Wirkstoffe können als solche, in Form von Präparaten oder in Gebrauchsformen, die daraus durch weitere Verdünnung hergestellt werden, eingesetzt werden, beispielsweise in Form gebrauchsfertiger Lösungen, Suspensionen, Emulsionen, Pulver, Pasten und als Granulat. Sie können in üblicher Weise angewandt werden, beispielsweise durch Bewässern, Sprühen, Zerstäuben, Streuen oder Stäuben.

Die Mengen der aktiven Verbindung in den gebrauchsfertigen Präparaten können, den Umständen entsprechend, innerhalb eines breiten Rahmens variiert werden. Sie betragen im allgemeinen von 0,01 bis 95 Gewichts-%, vorzugsweise von 0,05 bis 60 Gewichts-%.

Die Verbindungen können auch mittels des ULV-Verfahrens (ultra-low-volume method) zur Anwendung gebracht werden, wobei die verwendeten Präparate bis zu 100 % des Wirkstoffes enthalten können.

Die Wirkstoffe können nach dem Auflaufen der Pflanzen oder vor dem Auflaufen der Pflanzen d. h. mittels des Verfahrens der Vorauflaufbehandlung, zur Anwendung gelangen, angewandt werden. Sie können auch vor der Einsaat in den Boden eingearbeitet werden.

Die aufgewandten Wirkstoff-Mengen können innerhalb eines beträchtlichen Bereichs variiert werden. Sie hängen im wesentlichen von der Art der angestrebten Wirkung ab. Im allgemeinen liegen die flächenbezogenen Aufwandsmengen des Wirkstoffs zwischen 0,01 und 2 kg/ha, vorzugsweise zwischen 0,05 und 1 kg/ha.

Die vorliegende Erfindung umfaßt weiter ein herbizides Mittel, das als Wirkstoff eine Verbindung gemäß der vorliegenden Erfindung im Gemisch mit einem festen Verdünnungsmittel oder Träger oder im Gemisch mit einem ein oberflächenaktives Mittel enthaltenden flüssigen Verdünnungsmittel oder Träger enthalten.

Die vorliegende Erfindung umfaßt weiter ein Verfahren zur Unkrautbekämpfung, das dadurch gekennzeichnet ist, daß eine Verbindung gemäß der vorliegenden Erfindung allein oder in Form eines Präparats, das als Wirkstoff eine Verbindung gemäß der vorliegenden Erfindung im Gemisch mit einem Verdünnungsmittel oder Träger enthält, auf die Unkräuter oder deren Lebensraum aufgebracht wird.

Die vorliegende Erfindung ist ferner auf Nutzpflanzen gerichtet, die dadurch gegen Schäden durch Unkräuter geschützt sind, daß sie auf Flächen angebaut werden, auf die unmittelbar vor und/oder während der Zeit des Anbauens eine Verbindung gemäß der vorliegenden Erfindung allein oder im Gemisch mit einem Verdünnungsmittel oder Träger aufgebracht wurde.

Es wird ersichtlich werden, daß die üblichen Methoden der Erzeugung geernteter Nutzpflanzen durch die vorliegende Erfindung verbessert werden können.

Zusammensetzungen gemäß der vorliegenden Erfindung werden in den folgenden Beispielen erläutert. Hierin werden die Verbindungen der vorliegenden Erfindung durch die (in Klammer angegebene) Zahl des betreffenden Herstellungsbeispiels bezeichnet. Die Bezeichnung « Teile » bedeutet Gewichtsteile.

### Beispiel i

15 Teile der Verbindung (1), 80 Teile eines 1 : 5-Gemisches aus pulvriger Diatomeenerde und Tonpulver, 2 Teile Natriumalkylbenzolsulfonat und 3 Teile Natriumalkylnaphthalinsulfonat/Formaldehyd-Kondensat wurden pulverisiert und zu einem benetzbaren Pulver vermischt. Vor dem Gebrauch wurde das benetzbare Pulver mit Wasser verdünnt.

### Beispiel ii

30 Teile der Verbindung (11), 55 Teile Xylol, 8 Teile Polyoxyethylen-alkylphenylether und 7 Teile Calciumalkylbenzolsulfonat wurden unter Rühren miteinander vermischt, wodurch ein emulgierbares Konzentrat erhalten wurde. Vor dem Gebrauch wurde das emulgierbare Konzentrat mit Wasser verdünnt.

Beispiel iii

2 Teile der Verbindung (12) und 98 Teile Tonpulver wurden pulverisiert und gemischt, wodurch ein Stäubemittel erhalten wurde.

Beispiel iv

1,5 Teile der Verbindung (13), 0,5 Teile Isopropylhydrogenphosphat (PAP) und 98 Teile Tonpulver wurden pulverisiert und gemischt, wodurch ein Stäubemittel erhalten wurde.

Beispiel v

25 Teile Wasser wurden zu einer Mischung aus 10 Teilen der Verbindung (14), 30 Teilen Bentonit (Montmorillonit), 58 Teilen Talkum und 2 Teilen Ligninsulfonat-Salz zugesetzt, und das Gemisch wurde gut durchgemischt. Die erhaltene Mischung wurde mittels eines Extruder-Granulators zu einem Granulat mit einer Korngröße von 0,43 bis 2,0 mm (10 bis 40 mesh) verarbeitet und bei 40 °C bis 50 °C getrocknet, wodurch ein Granulat erhalten wurde.

Beispiel vi

Ein Drehmischer wurde mit 95 Teilen Tonmineral-Teilchen mit einer Teilchengrößen-Verteilung im Bereich von 0,2 bis 2 mm beschickt, und unter Drehen des Mischers wurden 5 Teile der Verbindung (2), gelöst in einem organischen Lösungsmittel, gleichmäßig auf die Tonmineral-Teilchen aufgesprüht. Die Teilchen wurden dann bei 40 °C bis 50 °C getrocknet, wodurch ein Granulat erhalten wurde.

Die herbizide Wirkung der Verbindungen der Formel (I) wird durch die folgenden Biotest-Beispiele aufgezeigt, in denen die Verbindungen der vorliegenden Erfindung durch die (in Klammer angegebene) Zahl des betreffenden Herstellungsbeispiels bezeichnet werden.

Die bekannten Vergleichsverbindungen werden durch die nachstehenden Formeln beschrieben :

(A-1) = 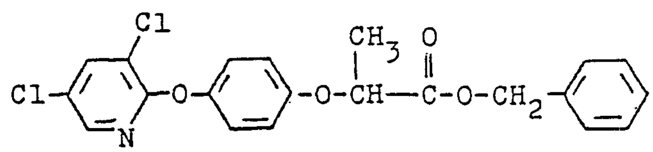

(A-2) = 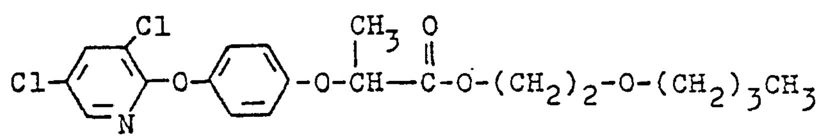

Diese Verbindungen wurden in der JP-OS 51-106 735 beschrieben.

(B-1) = 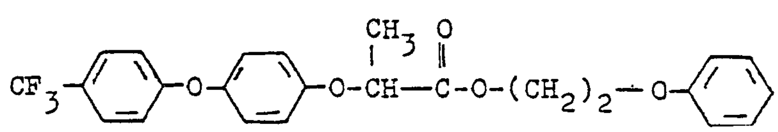

Diese Verbindung wurde in der JP-OS 52-131 545 beschrieben.

(C-1) = 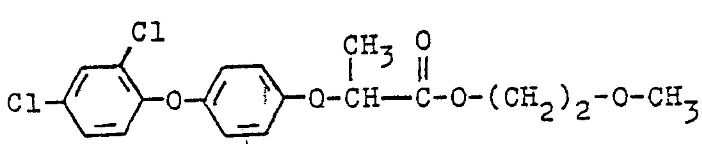

Diese Verbindung wurde in der JP-OS 52-144 637 beschrieben.

(D-1) = 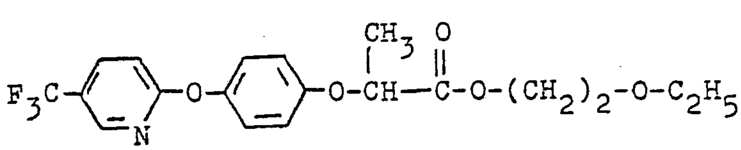

Diese Verbindung wurde in der JP-OS 54-119 476 beschrieben.

Beispiel A

Prüfung der Wirkung der Vorauflaufbehandlung auf Unkräuter und Nutzpflanzen auf höher gelegenen Feldern :

Herstellung eines Wirkstoffs :

Träger : 5 Gewichtsteile Aceton ;
Emulgator : 1 Gewichtsteil Benzyloxypolyglycolether.

Ein Wirkstoff-Präparat in Form eines emulgierbaren Konzentrats wurde durch Vermischen von 1 Teil der aktiven Verbindung mit den vorbezeichneten Mengen Träger und Emulgator erhalten. Eine vorbestimmte Menge des Präparats wurde durch Verdünnen mit Wasser erhalten.

Test-Verfahren :

In Ackerboden, der in Töpfe (1 000 $cm^2$) gefüllt worden war, wurden in einem Gewächshaus jeweils Arachis, Pisum, Gossypium und Glycine eingesät ; danach wurde mit einer 1 cm dicken Schicht eines Bodens, dem Samen von Agropyron repens, Echinochloa crus-galli und Setaria lutescens zugemischt waren, abgedeckt. Einen Tag nach der Einsaat wurden jeweils 10 ml der wie oben beschrieben hergestellten Lösungen, die 500 ppm, 200 ppm, 100 ppm bzw. 50 ppm des Wirkstoffs enthielten, gleichmäßig auf die Oberflächenschicht des Bodens aufgebracht.

Vier Wochen nach der Behandlung wurden die herbizide Wirkung und der Grad der Phytotoxizität mit Hilfe einer Skala von 0 bis 10 nach dem folgenden Maßstah bewertet.

Die herbizide Wirkung wurde im Vergleich zu unbehandelten Kontrollpflanzen wie folgt bewertet :

| Bewertung | Unkrautvernichtungsrate (bezogen auf die unbehandelten Kontrollpflanzen) |
|---|---|
| 10 | 100 % (verdorrt) |
| 9 | mindestens 90 %, jedoch weniger als 100 % |
| 8 | mindestens 80 %, jedoch weniger als 90 % |
| 7 | mindestens 70 %, jedoch weniger als 80 % |
| 6 | mindestens 60 %, jedoch weniger als 70 % |
| 5 | mindestens 50 %, jedoch weniger als 60 % |
| 4 | mindestens 40 %, jedoch weniger als 50 % |
| 3 | mindestens 30 %, jedoch weniger als 40 % |
| 2 | mindestens 20 %, jedoch weniger als 30 % |
| 1 | mindestens 10 %, jedoch weniger als 20 % |
| 0 | weniger als 10 % (unwirksam) |

Die Phytotoxizität gegenüber den Nutzpflanzen wurde im Vergleich zu den unbehandelten Kontrollpflanzen wie folgt bewertet :

| Bewertung | Phytotoxizitätsrate (bezogen auf die unbehandelten Kontrollpflanzen) |
|---|---|
| 10 | mindestens 90 % (Ausrottung) |
| 9 | mindestens 80 %, jedoch weniger als 90 % |
| 8 | mindestens 70 %, jedoch weniger als 80 % |
| 7 | mindestens 60 %, jedoch weniger als 70 % |
| 6 | mindestens 50 %, jedoch weniger als 60 % |
| 5 | mindestens 40 %, jedoch weniger als 50 % |
| 4 | mindestens 30 %, jedoch weniger als 40 % |
| 3 | mindestens 20 %, jedoch weniger als 30 % |
| 2 | mindestens 10 %, jedoch weniger als 20 % |
| 1 | mehr als 0 %, jedoch weniger als 10 % |
| 0 | 0 % (keine Phytotoxizität) |

Die Testergebnisse sind in Tabelle 1 aufgeführt, in der die Symbole a bis g die folgenden Unkräuter und Nutzpflanzen bezeichnen :

a : Agropyron repens,
b : Echinochloa crus-galli,
c : Setaria lutescens
d : Arachis,
e : Pisum
f : Gossypium,
g : Glycine.

Tabelle 1

| Verbindung Nr. | Wirkstoff-Menge kg/ha | Herbizide Wirkung Unkräuter a | b | c | Phytotoxizität Nutzpflanzen d | e | f | g |
|---|---|---|---|---|---|---|---|---|
| 1 | 0,5 | 10 | 10 | 10 | 0 | 0 | 0 | 0 |
| | 0,2 | 10 | 10 | 10 | 0 | 0 | 0 | 0 |
| | 0,1 | 8 | 9 | 10 | 0 | 0 | 0 | 0 |
| | 0,05 | 6 | 7 | 9 | 0 | 0 | 0 | 0 |
| 3 | 0,5 | 10 | 10 | 10 | 0 | 0 | 0 | 0 |
| | 0,2 | 10 | 10 | 10 | 0 | 0 | 0 | 0 |
| | 0,1 | 8 | 9 | 10 | 0 | 0 | 0 | 0 |
| | 0,05 | 6 | 7 | 9 | 0 | 0 | 0 | 0 |
| 4 | 0,5 | 10 | 10 | 10 | 0 | 0 | 0 | 0 |
| | 0,2 | 10 | 10 | 10 | 0 | 0 | 0 | 0 |
| | 0,1 | 8 | 9 | 9 | 0 | 0 | 0 | 0 |
| | 0,05 | 6 | 7 | 8 | 0 | 0 | 0 | 0 |
| 36 | 0,5 | 10 | 10 | 10 | 0 | 0 | 0 | 0 |
| | 0,2 | 10 | 9 | 10 | 0 | 0 | 0 | 0 |
| | 0,1 | 8 | 8 | 9 | 0 | 0 | 0 | 0 |
| | 0,05 | 6 | 7 | 8 | 0 | 0 | 0 | 0 |
| 37 | 0,5 | 10 | 10 | 10 | 0 | 0 | 0 | 0 |
| | 0,2 | 10 | 10 | 10 | 0 | 0 | 0 | 0 |
| | 0,1 | 8 | 9 | 10 | 0 | 0 | 0 | 0 |
| | 0,05 | 7 | 8 | 9 | 0 | 0 | 0 | 0 |
| 38 | 0,5 | 10 | 10 | 10 | 0 | 0 | 0 | 0 |
| | 0,2 | 10 | 10 | 10 | 0 | 0 | 0 | 0 |
| | 0,1 | 8 | 9 | 10 | 0 | 0 | 0 | 0 |
| | 0,05 | 7 | 8 | 10 | 0 | 0 | 0 | 0 |

Tabelle 1 — Fortsetzung

| Verbindung Nr. | Wirkstoff-Menge kg/ha | Herbizide Wirkung Unkräuter a | b | c | Phytotoxizität Nutzpflanzen d | e | f | g |
|---|---|---|---|---|---|---|---|---|
| 39 | 0,5 | 10 | 10 | 10 | 0 | 0 | 0 | 0 |
| | 0,2 | 10 | 10 | 10 | 0 | 0 | 0 | 0 |
| | 0,1 | 9 | 9 | 10 | 0 | 0 | 0 | 0 |
| | 0,05 | 8 | 8 | 9 | 0 | 0 | 0 | 0 |
| 29 | 0,5 | 10 | 10 | 10 | 0 | 0 | 0 | 0 |
| | 0,2 | 10 | 10 | 10 | 0 | 0 | 0 | 0 |
| | 0,1 | 10 | 10 | 10 | 0 | 0 | 0 | 0 |
| | 0,05 | 9 | 10 | 10 | 0 | 0 | 0 | 0 |
| 44 | 0,5 | 10 | 10 | 10 | 0 | 0 | 0 | 0 |
| | 0,2 | 10 | 10 | 10 | 0 | 0 | 0 | 0 |
| | 0,1 | 9 | 9 | 10 | 0 | 0 | 0 | 0 |
| | 0,05 | 8 | 7 | 9 | 0 | 0 | 0 | 0 |
| 14 | 0,5 | 10 | 10 | 10 | 0 | 0 | 0 | 0 |
| | 0,2 | 10 | 10 | 10 | 0 | 0 | 0 | 0 |
| | 0,1 | 8 | 8 | 9 | 0 | 0 | 0 | 0 |
| | 0,05 | 6 | 6 | 8 | 0 | 0 | 0 | 0 |
| 58 | 0,5 | 10 | 10 | 10 | 0 | 0 | 0 | 0 |
| | 0,2 | 10 | 10 | 10 | 0 | 0 | 0 | 0 |
| | 0,1 | 8 | 8 | 9 | 0 | 0 | 0 | 0 |
| | 0,05 | 6 | 6 | 8 | 0 | 0 | 0 | 0 |
| 63 | 0,5 | 10 | 10 | 10 | 0 | 0 | 0 | 0 |
| | 0,2 | 10 | 10 | 10 | 0 | 0 | 0 | 0 |
| | 0,1 | 10 | 10 | 10 | 0 | 0 | 0 | 0 |
| | 0,05 | 9 | 9 | 9 | 0 | 0 | 0 | 0 |
| 64 | 0,5 | 10 | 10 | 10 | 0 | 0 | 0 | 0 |
| | 0,2 | 10 | 10 | 10 | 0 | 0 | 0 | 0 |
| | 0,1 | 9 | 9 | 9 | 0 | 0 | 0 | 0 |
| | 0,05 | 8 | 8 | 9 | 0 | 0 | 0 | 0 |
| 65 | 0,5 | 10 | 10 | 10 | 0 | 0 | 0 | 0 |
| | 0,2 | 10 | 10 | 10 | 0 | 0 | 0 | 0 |
| | 0,1 | 9 | 9 | 9 | 0 | 0 | 0 | 0 |
| | 0,05 | 8 | 9 | 9 | 0 | 0 | 0 | 0 |

Tabelle 1 — Fortsetzung

| Verbindung Nr. | Wirkstoff-Menge kg/ha | Herbizide Wirkung Unkräuter a | b | c | Phytotoxizität Nutzpflanzen d | e | f | g |
|---|---|---|---|---|---|---|---|---|
| 20 | 0,5 | 10 | 10 | 10 | 0 | 0 | 0 | 0 |
| | 0,2 | 10 | 10 | 10 | 0 | 0 | 0 | 0 |
| | 0,1 | 9 | 9 | 9 | 0 | 0 | 0 | 0 |
| | 0,05 | 8 | 8 | 9 | 0 | 0 | 0 | 0 |
| 22 | 0,5 | 10 | 10 | 10 | 0 | 0 | 0 | 0 |
| | 0,2 | 10 | 10 | 10 | 0 | 0 | 0 | 0 |
| | 0,1 | 9 | 9 | 9 | 0 | 0 | 0 | 0 |
| | 0,05 | 8 | 8 | 9 | 0 | 0 | 0 | 0 |
| 24 | 0,5 | 10 | 10 | 10 | 0 | 0 | 0 | 0 |
| | 0,2 | 10 | 10 | 10 | 0 | 0 | 0 | 0 |
| | 0,1 | 9 | 9 | 9 | 0 | 0 | 0 | 0 |
| | 0,05 | 8 | 8 | 8 | 0 | 0 | 0 | 0 |
| 72 | 0,5 | 10 | 10 | 10 | 0 | 0 | 0 | 0 |
| | 0,2 | 9 | 10 | 9 | 0 | 0 | 0 | 0 |
| | 0,1 | 8 | 9 | 9 | 0 | 0 | 0 | 0 |
| | 0,05 | 7 | 8 | 8 | 0 | 0 | 0 | 0 |
| 74 | 0,5 | 10 | 10 | 10 | 0 | 0 | 0 | 0 |
| | 0,2 | 10 | 10 | 10 | 0 | 0 | 0 | 0 |
| | 0,1 | 9 | 9 | 9 | 0 | 0 | 0 | 0 |
| | 0,05 | 8 | 9 | 9 | 0 | 0 | 0 | 0 |
| Vergl.- | 0,5 | 7 | 9 | 9 | 0 | 0 | 0 | 0 |
| Verb. | 0,2 | 5 | 7 | 8 | 0 | 0 | 0 | 0 |
| A-1 | 0,1 | 2 | 5 | 6 | 0 | 0 | 0 | 0 |
| | 0,05 | 0 | 2 | 4 | 0 | 0 | 0 | 0 |
| Vergl.- | 0,5 | 9 | 10 | 10 | 0 | 0 | 0 | 0 |
| Verb. | 0,2 | 7 | 8 | 8 | 0 | 0 | 0 | 0 |
| B-1 | 0,1 | 3 | 6 | 7 | 0 | 0 | 0 | 0 |
| | 0,05 | 0 | 3 | 4 | 0 | 0 | 0 | 0 |

Beispiel B

Prüfung der Wirkung der Laubbehandlung auf Unkräuter und Nutzpflanzen auf höher gelegenen Feldern :

In Ackerboden, der in Töpfe (2 000 $cm^2$) gefüllt worden war, wurden jeweils Glycine, Rephanus und Beta eingesät ; danach wurde mit einer 1 cm dicken Schicht eines Bodens, dem Samen von Echinochloa crus-galli, Digitaria adscendens, Eleusine indica, Setaria viridis, Avena fatua und Alopecurus aequalis zugemischt waren, abgedeckt. 10 Tage nach der Einsaat (wenn die Unkräuter sich im Durchschnitt im 2-Blatt-Stadium befanden und Glycine, Rephanus und Beta sich im Anfangsstadium der normalen Blatt-Wachstumsperiode befanden), wurden jeweils 20 ml der Lösungen, die wie in Test-Beispiel 1 hergestellt worden waren und 500 ppm, 200 ppm, 100 ppm bzw. 50 ppm des Wirkstoffs enthielten, gleichmäßig auf die Blätter der zu testenden Pflanzen aufgebracht.

Drei Wochen nach der Behandlung wurden die herbizide Wirkung und der Grad der Phytotoxizität in der gleichen Weise wie in Beispiel A bewertet.

Die Testergebnisse sind in Tabelle 2 aufgeführt, in der die Symbole h bis p die folgenden Unkräuter und Nutzpflanzen bezeichnen :

h : Echinochloa crus-galli,
i : Digitaria adscendens,
j : Eleusine indica,
k : Setaria viridis,
l : Avena fatua,
m : Aleopecurus aequalis,
n : Glycine,
o : Rephanus,
p : Beta.

Tabelle 2

| Verbindung Nr. | Wirkstoff-Menge kg/ha | Herbizide Wirkung Unkräuter h | i | j | k | l | m | Phytotoxizität Nutzpflanzen n | o | p |
|---|---|---|---|---|---|---|---|---|---|---|
| 1 | 0,5 | 10 | 10 | 10 | 10 | 10 | 10 | 0 | 0 | 0 |
| | 0,2 | 10 | 10 | 10 | 10 | 10 | 10 | 0 | 0 | 0 |
| | 0,1 | 10 | 10 | 10 | 10 | 10 | 10 | 0 | 0 | 0 |
| | 0,05 | 10 | 10 | 10 | 10 | 10 | 10 | 0 | 0 | 0 |
| 2 | 0,5 | 10 | 10 | 10 | 10 | 10 | 10 | 0 | 0 | 0 |
| | 0,2 | 10 | 10 | 10 | 10 | 10 | 10 | 0 | 0 | 0 |
| | 0,1 | 10 | 10 | 10 | 10 | 10 | 10 | 0 | 0 | 0 |
| | 0,05 | 10 | 10 | 10 | 10 | 10 | 10 | 0 | 0 | 0 |
| 3 | 0,5 | 10 | 10 | 10 | 10 | 10 | 10 | 0 | 0 | 0 |
| | 0,2 | 10 | 10 | 10 | 10 | 10 | 10 | 0 | 0 | 0 |
| | 0,1 | 10 | 10 | 10 | 10 | 10 | 10 | 0 | 0 | 0 |
| | 0,05 | 10 | 10 | 10 | 10 | 9 | 10 | 0 | 0 | 0 |
| 4 | 0,5 | 10 | 10 | 10 | 10 | 10 | 10 | 0 | 0 | 0 |
| | 0,2 | 10 | 10 | 10 | 10 | 10 | 10 | 0 | 0 | 0 |
| | 0,1 | 10 | 9 | 9 | 9 | 9 | 10 | 0 | 0 | 0 |
| | 0,05 | 9 | 9 | 9 | 9 | 8 | 9 | 0 | 0 | 0 |

Tabelle 2 — Fortsetzung

| Verbindung Nr. | Wirkstoff-Menge kg/ha | Herbizide Wirkung Unkräuter h | i | j | k | l | m | Phytotoxizität Nutzpflanzen n | o | p |
|---|---|---|---|---|---|---|---|---|---|---|
| 37 | 0,5 | 10 | 10 | 10 | 10 | 10 | 10 | 0 | 0 | 0 |
| | 0,2 | 10 | 10 | 10 | 10 | 10 | 10 | 0 | 0 | 0 |
| | 0,1 | 10 | 10 | 10 | 10 | 10 | 10 | 0 | 0 | 0 |
| | 0,05 | 10 | 10 | 10 | 10 | 10 | 10 | 0 | 0 | 0 |
| 38 | 0,5 | 10 | 10 | 10 | 10 | 10 | 10 | 0 | 0 | 0 |
| | 0,2 | 10 | 10 | 10 | 10 | 10 | 10 | 0 | 0 | 0 |
| | 0,1 | 10 | 10 | 10 | 10 | 10 | 10 | 0 | 0 | 0 |
| | 0,05 | 10 | 10 | 10 | 10 | 10 | 10 | 0 | 0 | 0 |
| 39 | 0,5 | 10 | 10 | 10 | 10 | 10 | 10 | 0 | 0 | 0 |
| | 0,2 | 10 | 10 | 10 | 10 | 10 | 10 | 0 | 0 | 0 |
| | 0,1 | 10 | 10 | 10 | 10 | 10 | 10 | 0 | 0 | 0 |
| | 0,05 | 10 | 10 | 10 | 10 | 10 | 10 | 0 | 0 | 0 |
| 29 | 0,5 | 10 | 10 | 10 | 10 | 10 | 10 | 0 | 0 | 0 |
| | 0,2 | 10 | 10 | 10 | 10 | 10 | 10 | 0 | 0 | 0 |
| | 0,1 | 10 | 10 | 10 | 10 | 10 | 10 | 0 | 0 | 0 |
| | 0,05 | 10 | 10 | 10 | 10 | 10 | 10 | 0 | 0 | 0 |
| 41 | 0,5 | 10 | 10 | 10 | 10 | 10 | 10 | 0 | 0 | 0 |
| | 0,2 | 10 | 10 | 10 | 10 | 10 | 10 | 0 | 0 | 0 |
| | 0,1 | 10 | 10 | 10 | 10 | 10 | 10 | 0 | 0 | 0 |
| | 0,05 | 10 | 10 | 10 | 10 | 10 | 10 | 0 | 0 | 0 |
| 42 | 0,5 | 10 | 10 | 10 | 10 | 10 | 10 | 0 | 0 | 0 |
| | 0,2 | 10 | 10 | 10 | 10 | 10 | 10 | 0 | 0 | 0 |
| | 0,1 | 10 | 10 | 9 | 9 | 8 | 9 | 0 | 0 | 0 |
| | 0,05 | 9 | 9 | 8 | 8 | 7 | 8 | 0 | 0 | 0 |
| 44 | 0,5 | 10 | 10 | 10 | 10 | 10 | 10 | 0 | 0 | 0 |
| | 0,2 | 10 | 10 | 10 | 10 | 10 | 10 | 0 | 0 | 0 |
| | 0,1 | 10 | 10 | 10 | 10 | 10 | 10 | 0 | 0 | 0 |
| | 0,05 | 10 | 10 | 9 | 9 | 9 | 9 | 0 | 0 | 0 |
| 48 | 0,5 | 10 | 10 | 10 | 10 | 10 | 10 | 0 | 0 | 0 |
| | 0,2 | 10 | 10 | 10 | 10 | 10 | 10 | 0 | 0 | 0 |
| | 0,1 | 10 | 10 | 10 | 9 | 9 | 10 | 0 | 0 | 0 |
| | 0,05 | 9 | 10 | 9 | 8 | 7 | 9 | 0 | 0 | 0 |

Tabelle 2 — Fortsetzung

| Verbindung | Wirkstoff-Menge | Herbizide Wirkung Unkräuter | | | | | | Phytotoxizität Nutzpflanzen | | |
|---|---|---|---|---|---|---|---|---|---|---|
| Nr. | kg/ha | h | i | j | k | l | m | n | o | p |
| 49 | 0,5 | 10 | 10 | 10 | 10 | 10 | 10 | 0 | 0 | 0 |
| | 0,2 | 10 | 10 | 10 | 10 | 10 | 10 | 0 | 0 | 0 |
| | 0,1 | 10 | 10 | 9 | 9 | 8 | 10 | 0 | 0 | 0 |
| | 0,05 | 9 | 9 | 8 | 8 | 7 | 9 | 0 | 0 | 0 |
| 50 | 0,5 | 10 | 10 | 10 | 10 | 10 | 10 | 0 | 0 | 0 |
| | 0,2 | 10 | 10 | 10 | 10 | 10 | 10 | 0 | 0 | 0 |
| | 0,1 | 10 | 10 | 10 | 10 | 10 | 10 | 0 | 0 | 0 |
| | 0,05 | 10 | 10 | 10 | 10 | 10 | 10 | 0 | 0 | 0 |
| 51 | 0,5 | 10 | 10 | 10 | 10 | 10 | 10 | 0 | 0 | 0 |
| | 0,2 | 10 | 10 | 10 | 10 | 10 | 10 | 0 | 0 | 0 |
| | 0,1 | 10 | 10 | 10 | 10 | 9 | 10 | 0 | 0 | 0 |
| | 0,05 | 9 | 10 | 9 | 9 | 8 | 9 | 0 | 0 | 0 |
| 52 | 0,5 | 10 | 10 | 10 | 10 | 10 | 10 | 0 | 0 | 0 |
| | 0,2 | 10 | 10 | 10 | 10 | 10 | 10 | 0 | 0 | 0 |
| | 0,1 | 10 | 10 | 10 | 10 | 10 | 10 | 0 | 0 | 0 |
| | 0,05 | 10 | 10 | 10 | 10 | 9 | 10 | 0 | 0 | 0 |
| 55 | 0,5 | 10 | 10 | 10 | 10 | 10 | 10 | 0 | 0 | 0 |
| | 0,2 | 10 | 10 | 10 | 10 | 10 | 10 | 0 | 0 | 0 |
| | 0,1 | 10 | 10 | 10 | 10 | 10 | 10 | 0 | 0 | 0 |
| | 0,05 | 10 | 10 | 9 | 9 | 9 | 9 | 0 | 0 | 0 |
| 57 | 0,5 | 10 | 10 | 10 | 10 | 10 | 10 | 0 | 0 | 0 |
| | 0,2 | 10 | 10 | 10 | 10 | 10 | 10 | 0 | 0 | 0 |
| | 0,1 | 10 | 10 | 9 | 9 | 9 | 10 | 0 | 0 | 0 |
| | 0,05 | 9 | 9 | 8 | 8 | 8 | 9 | 0 | 0 | 0 |
| 58 | 0,5 | 10 | 10 | 10 | 10 | 10 | 10 | 0 | 0 | 0 |
| | 0,2 | 10 | 10 | 10 | 10 | 10 | 10 | 0 | 0 | 0 |
| | 0,1 | 10 | 9 | 9 | 9 | 9 | 10 | 0 | 0 | 0 |
| | 0,05 | 9 | 8 | 8 | 9 | 7 | 9 | 0 | 0 | 0 |
| 59 | 0,5 | 10 | 10 | 10 | 10 | 10 | 10 | 0 | 0 | 0 |
| | 0,2 | 10 | 10 | 10 | 10 | 10 | 10 | 0 | 0 | 0 |
| | 0,1 | 10 | 10 | 10 | 10 | 10 | 10 | 0 | 0 | 0 |
| | 0,05 | 10 | 9 | 10 | 9 | 9 | 9 | 0 | 0 | 0 |

Tabelle 2 — Fortsetzung

| Verbindung Nr. | Wirkstoff-Menge kg/ha | Herbizide Wirkung Unkräuter h | i | j | k | l | m | Phytotoxizität Nutzpflanzen n | o | p |
|---|---|---|---|---|---|---|---|---|---|---|
| 16 | 0,5 | 10 | 10 | 10 | 10 | 10 | 10 | 0 | 0 | 0 |
| | 0,2 | 10 | 10 | 10 | 10 | 10 | 10 | 0 | 0 | 0 |
| | 0,1 | 10 | 10 | 10 | 10 | 10 | 10 | 0 | 0 | 0 |
| | 0,05 | 10 | 10 | 9 | 9 | 9 | 10 | 0 | 0 | 0 |
| 60 | 0,5 | 10 | 10 | 10 | 10 | 10 | 10 | 0 | 0 | 0 |
| | 0,2 | 10 | 10 | 10 | 10 | 10 | 10 | 0 | 0 | 0 |
| | 0,1 | 10 | 9 | 10 | 9 | 9 | 9 | 0 | 0 | 0 |
| | 0,05 | 9 | 7 | 9 | 9 | 8 | 9 | 0 | 0 | 0 |
| 61 | 0,5 | 10 | 10 | 10 | 10 | 10 | 10 | 0 | 0 | 0 |
| | 0,2 | 10 | 10 | 10 | 10 | 10 | 10 | 0 | 0 | 0 |
| | 0,1 | 9 | 9 | 9 | 9 | 9 | 10 | 0 | 0 | 0 |
| | 0,05 | 9 | 8 | 8 | 8 | 8 | 9 | 0 | 0 | 0 |
| 63 | 0,5 | 10 | 10 | 10 | 10 | 10 | 10 | 0 | 0 | 0 |
| | 0,2 | 10 | 10 | 10 | 10 | 10 | 10 | 0 | 0 | 0 |
| | 0,1 | 10 | 9 | 10 | 10 | 9 | 9 | 0 | 0 | 0 |
| | 0,05 | 9 | 8 | 9 | 9 | 9 | 9 | 0 | 0 | 0 |
| 17 | 0,5 | 10 | 10 | 10 | 10 | 10 | 10 | 0 | 0 | 0 |
| | 0,2 | 10 | 10 | 10 | 10 | 10 | 10 | 0 | 0 | 0 |
| | 0,1 | 10 | 9 | 10 | 10 | 10 | 10 | 0 | 0 | 0 |
| | 0,05 | 9 | 8 | 9 | 9 | 9 | 9 | 0 | 0 | 0 |
| 64 | 0,5 | 10 | 10 | 10 | 10 | 10 | 10 | 0 | 0 | 0 |
| | 0,2 | 10 | 10 | 10 | 10 | 10 | 10 | 0 | 0 | 0 |
| | 0,1 | 10 | 9 | 10 | 10 | 10 | 9 | 0 | 0 | 0 |
| | 0,05 | 10 | 8 | 9 | 9 | 9 | 9 | 0 | 0 | 0 |
| 65 | 0,5 | 10 | 10 | 10 | 10 | 10 | 10 | 0 | 0 | 0 |
| | 0,2 | 10 | 10 | 10 | 10 | 10 | 10 | 0 | 0 | 0 |
| | 0,1 | 10 | 8 | 10 | 10 | 9 | 9 | 0 | 0 | 0 |
| | 0,05 | 9 | 7 | 9 | 9 | 8 | 8 | 0 | 0 | 0 |
| 67 | 0,5 | 10 | 10 | 10 | 10 | 10 | 10 | 0 | 0 | 0 |
| | 0,2 | 10 | 10 | 10 | 10 | 10 | 10 | 0 | 0 | 0 |
| | 0,1 | 10 | 8 | 10 | 9 | 9 | 9 | 0 | 0 | 0 |
| | 0,05 | 9 | 7 | 9 | 9 | 8 | 8 | 0 | 0 | 0 |

Tabelle 2 — Fortsetzung

| Verbindung Nr. | Wirkstoff-Menge kg/ha | Herbizide Wirkung Unkräuter h | i | j | k | l | m | Phytotoxizität Nutzpflanzen n | o | p |
|---|---|---|---|---|---|---|---|---|---|---|
| 68 | 0,5 | 10 | 10 | 10 | 10 | 10 | 10 | 0 | 0 | 0 |
| | 0,2 | 10 | 10 | 10 | 10 | 10 | 10 | 0 | 0 | 0 |
| | 0,1 | 10 | 10 | 10 | 10 | 9 | 10 | 0 | 0 | 0 |
| | 0,05 | 10 | 9 | 9 | 9 | 9 | 9 | 0 | 0 | 0 |
| 20 | 0,5 | 10 | 10 | 10 | 10 | 10 | 10 | 0 | 0 | 0 |
| | 0,2 | 10 | 10 | 10 | 10 | 10 | 10 | 0 | 0 | 0 |
| | 0,1 | 10 | 10 | 10 | 10 | 10 | 10 | 0 | 0 | 0 |
| | 0,05 | 10 | 9 | 9 | 9 | 9 | 9 | 0 | 0 | 0 |
| 21 | 0,5 | 10 | 10 | 10 | 10 | 10 | 10 | 0 | 0 | 0 |
| | 0,2 | 10 | 10 | 10 | 10 | 10 | 10 | 0 | 0 | 0 |
| | 0,1 | 10 | 10 | 10 | 10 | 10 | 10 | 0 | 0 | 0 |
| | 0,05 | 10 | 9 | 9 | 9 | 9 | 10 | 0 | 0 | 0 |
| 70 | 0,5 | 10 | 10 | 10 | 10 | 10 | 10 | 0 | 0 | 0 |
| | 0,2 | 10 | 10 | 10 | 10 | 10 | 10 | 0 | 0 | 0 |
| | 0,1 | 10 | 10 | 9 | 10 | 10 | 10 | 0 | 0 | 0 |
| | 0,05 | 9 | 9 | 9 | 9 | 9 | 9 | 0 | 0 | 0 |
| 71 | 0,5 | 10 | 10 | 10 | 10 | 10 | 10 | 0 | 0 | 0 |
| | 0,2 | 10 | 10 | 10 | 10 | 10 | 10 | 0 | 0 | 0 |
| | 0,1 | 10 | 9 | 9 | 10 | 9 | 9 | 0 | 0 | 0 |
| | 0,05 | 9 | 9 | 9 | 9 | 9 | 9 | 0 | 0 | 0 |
| 24 | 0,5 | 10 | 10 | 10 | 10 | 10 | 10 | 0 | 0 | 0 |
| | 0,2 | 10 | 10 | 10 | 10 | 10 | 10 | 0 | 0 | 0 |
| | 0,1 | 10 | 10 | 10 | 10 | 10 | 10 | 0 | 0 | 0 |
| | 0,05 | 10 | 9 | 9 | 9 | 9 | 10 | 0 | 0 | 0 |
| 25 | 0,5 | 10 | 10 | 10 | 10 | 10 | 10 | 0 | 0 | 0 |
| | 0,2 | 10 | 10 | 10 | 10 | 10 | 10 | 0 | 0 | 0 |
| | 0,1 | 10 | 10 | 10 | 10 | 10 | 10 | 0 | 0 | 0 |
| | 0,05 | 10 | 9 | 9 | 9 | 9 | 10 | 0 | 0 | 0 |

Tabelle 2 — Fortsetzung

| Verbindung Nr. | Wirkstoff-Menge kg/ha | Herbizide Wirkung Unkräuter h | i | j | k | l | m | Phytotoxizität Nutzpflanzen n | o | p |
|---|---|---|---|---|---|---|---|---|---|---|
| 72 | 0,5 | 10 | 10 | 10 | 10 | 10 | 10 | 0 | 0 | 0 |
| | 0,2 | 10 | 10 | 10 | 10 | 10 | 10 | 0 | 0 | 0 |
| | 0,1 | 10 | 10 | 10 | 10 | 10 | 10 | 0 | 0 | 0 |
| | 0,05 | 10 | 9 | 10 | 10 | 9 | 9 | 0 | 0 | 0 |
| 27 | 0,5 | 10 | 10 | 10 | 10 | 10 | 10 | 0 | 0 | 0 |
| | 0,2 | 10 | 10 | 10 | 10 | 10 | 10 | 0 | 0 | 0 |
| | 0,1 | 10 | 10 | 10 | 10 | 10 | 10 | 0 | 0 | 0 |
| | 0,05 | 10 | 10 | 10 | 10 | 9 | 9 | 0 | 0 | 0 |
| 73 | 0,5 | 10 | 10 | 10 | 10 | 10 | 10 | 0 | 0 | 0 |
| | 0,2 | 10 | 10 | 10 | 10 | 10 | 10 | 0 | 0 | 0 |
| | 0,1 | 10 | 10 | 10 | 9 | 10 | 9 | 0 | 0 | 0 |
| | 0,05 | 10 | 9 | 9 | 9 | 9 | 9 | 0 | 0 | 0 |
| 23 | 0,5 | 10 | 10 | 10 | 10 | 10 | 10 | 0 | 0 | 0 |
| | 0,2 | 10 | 10 | 10 | 10 | 10 | 10 | 0 | 0 | 0 |
| | 0,1 | 10 | 10 | 10 | 10 | 10 | 10 | 0 | 0 | 0 |
| | 0,05 | 10 | 9 | 9 | 9 | 9 | 9 | 0 | 0 | 0 |
| 76 | 0,5 | 10 | 10 | 10 | 10 | 10 | 10 | 0 | 0 | 0 |
| | 0,2 | 10 | 10 | 10 | 10 | 10 | 10 | 0 | 0 | 0 |
| | 0,1 | 10 | 10 | 10 | 10 | 10 | 10 | 0 | 0 | 0 |
| | 0,05 | 10 | 9 | 9 | 9 | 9 | 9 | 0 | 0 | 0 |
| 78 | 0,5 | 10 | 10 | 10 | 10 | 10 | 10 | 0 | 0 | 0 |
| | 0,2 | 10 | 10 | 10 | 10 | 10 | 10 | 0 | 0 | 0 |
| | 0,1 | 10 | 10 | 10 | 10 | 10 | 10 | 0 | 0 | 0 |
| | 0,05 | 10 | 9 | 9 | 9 | 9 | 9 | 0 | 0 | 0 |
| Vergl.-Verb. | | | | | | | | | | |
| A-2 | 0,5 | 10 | 10 | 10 | 10 | 9 | 10 | 0 | 0 | 0 |
| | 0,2 | 9 | 10 | 9 | 9 | 7 | 10 | 0 | 0 | 0 |
| | 0,1 | 7 | 8 | 6 | 7 | 5 | 8 | 0 | 0 | 0 |
| | 0,05 | 5 | 5 | 5 | 6 | 4 | 6 | 0 | 0 | 0 |

Tabelle 2 — Fortsetzung

| Verbindung Nr. | Wirkstoff-Menge kg/ha | Herbizide Wirkung Unkräuter h | i | j | k | l | m | Phytotoxizität Nutzpflanzen n | o | p |
|---|---|---|---|---|---|---|---|---|---|---|
| C-1 | 0,5 | 10 | 10 | 9 | 10 | 9 | 10 | 0 | 0 | 0 |
| | 0,2 | 10 | 9 | 6 | 9 | 6 | 9 | 0 | 0 | 0 |
| | 0,1 | 7 | 5 | 3 | 6 | 3 | 6 | 0 | 0 | 0 |
| | 0,05 | 3 | 2 | 1 | 3 | 1 | 3 | 0 | 0 | 0 |

## Beispiel C

Prüfung der herbiziden Wirkung und der Bekämpfungswirkung gegen das Nachwachsen bei Agropyron tsukushiense :

Reisfelder, auf denen Agropyron tsukushiense verbreitet in Büscheln auftrat, wurden in Parzellen von 1 $m^2$ Größe unterteilt. Jeweils 100 ml der Lösungen, die wie in Test-Beispiel 1 hergestellt worden waren und 1 000 ppm, 500 ppm, 200 ppm bzw. 100 ppm des Wirkstoffs enthielten, wurden in jeder Parzelle gleichmäßig auf das Laub von Agropyron tsukushiense aufgebracht. Die herbizide Wirkung wurde 20 Tage nach der Behandlung in gleicher Weise wie in Beispiel A bewertet. Weiterhin wurde die Bekämpfungswirkung gegen das Nachwachsen von Agropyron tsukushiense 40 und 60 Tage nach der Behandlung nach dem folgenden Maßstab bewertet :

| Bewertung | Rate der Bekämpfungswirkung gegen das Nachwachsen (bezogen auf die unbehandelten Kontrollpflanzen) |
|---|---|
| 10 | 100 % (vollständige Bekämpfung des Nachwachsens) |
| 9 | mindestens 90 %, jedoch weniger als 100 % |
| 8 | mindestens 80 %, jedoch weniger als 90 % |
| 7 | mindestens 70 %, jedoch weniger als 80 % |
| 6 | mindestens 60 %, jedoch weniger als 70 % |
| 5 | mindestens 50 %, jedoch weniger als 60 % |
| 4 | mindestens 40 %, jedoch weniger als 50 % |
| 3 | mindestens 30 %, jedoch weniger als 40 % |
| 2 | mindestens 20 %, jedoch weniger als 30 % |
| 1 | mindestens 10 %, jedoch weniger als 20 % |
| 0 | weniger als 10 % (keine Bekämpfungswirkung gegen das Nachwachsen) |

Die Testergebnisse sind in Tabelle 3 aufgeführt.

Tabelle 3

| Verbindung Nr. | Wirkstoff-Menge kg/ha | Herbizide Wirkung 20 Tage nach der Behandl. | Bekämpfungswirkung gegen Nachwachsen 40 Tage nach der Behandl. | 60 Tage nach der Behandl. |
|---|---|---|---|---|
| 1 | 1,0 | 10 | 10 | 10 |
| | 0,5 | 10 | 10 | 10 |
| | 0,2 | 10 | 10 | 10 |
| | 0,1 | 9 | 8 | 7 |
| 29 | 1,0 | 10 | 10 | 10 |
| | 0,5 | 10 | 10 | 10 |
| | 0,2 | 10 | 10 | 10 |
| | 0,1 | 9 | 8 | 7 |
| 58 | 1,0 | 10 | 10 | 10 |
| | 0,5 | 10 | 10 | 10 |
| | 0,2 | 10 | 10 | 9 |
| | 0,1 | 10 | 9 | 8 |
| 20 | 1,0 | 10 | 10 | 10 |
| | 0,5 | 10 | 10 | 10 |
| | 0,2 | 10 | 10 | 10 |
| | 0,1 | 9 | 9 | 8 |
| 21 | 1,0 | 10 | 10 | 10 |
| | 0,5 | 10 | 10 | 10 |
| | 0,2 | 10 | 10 | 10 |
| | 0,1 | 9 | 8 | 8 |
| 24 | 1,0 | 10 | 10 | 10 |
| | 0,5 | 10 | 10 | 10 |
| | 0,2 | 10. | 10 | 10 |
| | 0,1 | 9 | 8 | 7 |
| 74 | 1,0 | 10 | 10 | 10 |
| | 0,5 | 10 | 10 | 10 |
| | 0,2 | 10 | 10 | 9 |
| | 0,1 | 9 | 9 | 8 |
| 77 | 1,0 | 10 | 10 | 10 |
| | 0,5 | 10 | 10 | 10 |
| | 0,2 | 10 | 10 | 10 |
| | 0,1 | 9 | 8 | 7 |

Tabelle 3 — Fortsetzung

| Verbindung Nr. | Wirkstoff-Menge kg/ha | Herbizide Wirkung 20 Tage nach der Behandl. | Bekämpfungswirkung gegen Nachwachsen 40 Tage nach der Behandl. | 60 Tage nach der Behandl. |
|---|---|---|---|---|
| Vergl.- | 1,0 | 10 | 10 | 6 |
| Verb. | 0,5 | 7 | 6 | 3 |
| B-1 | 0,2 | 4 | 3 | 0 |
| | 0,1 | 1 | 0 | 0 |
| Vergl.- | 1,0 | 10 | 10 | 7 |
| Verb. | 0,5 | 9 | 7 | 2 |
| D-1 | 0,2 | 6 | 3 | 0 |
| | 0,1 | 3 | 0 | 0 |

Beispiel D

Prüfung der herbiziden Wirkung und der Bekämpfungswirkung gegen das Nachwachsen bei Cynodon dactylon :

Höher gelegene Felder, auf denen Cynodon dactylon verbreitet in Büscheln auftrat, wurden in Parzellen von 1 $m^2$ Größe unterteilt. Der Wirkstoff wurde in den gleichen Mengen wie in Beispiel C zur Behandlung von Cynodon dactylon angewandt.

In gleicher Weise wie in den vorhergehenden Beispielen A bis C wurden die herbizide Wirkung 20 Tage nach der Behandlung und die Bekämpfungswirkung gegen das Nachwachsen 40 und 60 Tage nach der Behandlung bewertet.

Die Testergebnisse sind in Tabelle 4 aufgeführt.

Tabelle 4

| Verbindung Nr. | Wirkstoff-Menge kg/ha | Herbizide Wirkung 20 Tage nach der Behandl. | Bekämpfungswirkung gegen Nachwachsen 40 Tage nach der Behandl. | 60 Tage nach der Behandl. |
|---|---|---|---|---|
| 1 | 1,0 | 10 | 10 | 10 |
| | 0,5 | 10 | 10 | 10 |
| | 0,2 | 10 | 10 | 10 |
| | 0,1 | 8 | 7 | 5 |
| 2 | 1,0 | 10 | 10 | 10 |
| | 0,5 | 10 | 10 | 10 |
| | 0,2 | 10 | 10 | 8 |
| | 0,1 | 10 | 8 | 7 |

Tabelle 4 — Fortsetzung

| Verbindung Nr. | Wirkstoff-Menge kg/ha | Herbizide Wirkung 20 Tage nach der Behandl. | Bekämpfungswirkung gegen Nachwachsen 40 Tage nach der Behandl. | 60 Tage nach der Behandl. |
|---|---|---|---|---|
| 4 | 1,0 | 10 | 10 | 10 |
| | 0,5 | 10 | 10 | 10 |
| | 0,2 | 10 | 10 | 9 |
| | 0,1 | 9 | 8 | 6 |
| 37 | 1,0 | 10 | 10 | 10 |
| | 0,5 | 10 | 10 | 10 |
| | 0,2 | 10 | 10 | 8 |
| | 0,1 | 9 | 8 | 7 |
| 20 | 1,0 | 10 | 10 | 10 |
| | 0,5 | 10 | 10 | 10 |
| | 0,2 | 10 | 9 | 8 |
| | 0,1 | 9 | 9 | 7 |
| 21 | 1,0 | 10 | 10 | 10 |
| | 0,5 | 10 | 10 | 10 |
| | 0,2 | 10 | 9 | 8 |
| | 0,1 | 9 | 8 | 7 |
| 75 | 1,0 | 10 | 10 | 10 |
| | 0,5 | 10 | 10 | 10 |
| | 0,2 | 10 | 9 | 8 |
| | 0,1 | 9 | 8 | 7 |
| 79 | 1,0 | 10 | 10 | 10 |
| | 0,5 | 10 | 10 | 10 |
| | 0,2 | 10 | 9 | 8 |
| | 0,1 | 9 | 7 | 6 |
| Vergl.- | 1,0 | 10 | 9 | 5 |
| Verb. | 0,5 | 8 | 5 | 2 |
| B-1 | 0,2 | 5 | 2 | 0 |
| | 0,1 | 4 | 0 | 0 |
| Vergl.- | 1,0 | 10 | 10 | 7 |
| Verb. | 0,5 | 9 | 7 | 5 |
| D-1 | 0,2 | 7 | 3 | 0 |
| | 0,1 | 5 | 1 | 0 |

Auch für andere als die in den vorhergehenden Beispielen A bis D eingesetzten Verbindungen wurde bestätigt, daß sie in den gleichen niedrigen Mengen, wie sie in den Beispielen A bis D verwendet wurden, hervorragende herbizide Wirkung besitzen und daß sie auch über eine lange Zeitdauer eine Bekämpfungswirkung gegen die Regeneration perennierender grasartiger Unkräuter zeigen.

Die folgenden Beispiele dienen der Erläuterung von Verfahren zur Herstellung von Verbindungen gemäß der vorliegenden Erfindung.

## Herstellungsbeispiele

### Beispiel 1

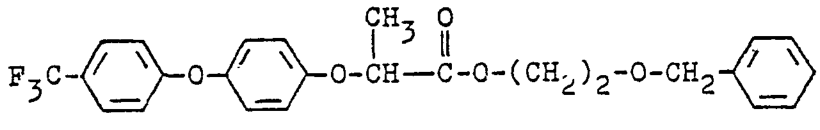

(1)

25,4 g 4-(4-Trifluoromethylphenoxy) phenol wurden zu 150 ml trockenem Acetonitril gegeben, und 28,7 g 2-Benzyloxyethyl-2-bromopropionat und 14,5 g wasserfreies Kaliumcarbonat wurden hinzugefügt. Die Mischung wurde anschließend unter gutem Durchrühren 4 h zum Rückfluß erhitzt. Nach Beendigung der Reaktion wurde das Acetonitril unter vermindertem Druck abdestilliert.

Dem Rückstand wurde Toluol zugesetzt, und die Toluol-Schicht wurde nacheinander mit einer wäßrigen Natrium-hydroxid-Lösung einer Stärke von 1 Gew.-% und mit Wasser gewaschen. Nach dem Abdestillieren des Toluols unter vermindertem Druck wurden als Endprodukt 41 g farbloses viskoses 2-Benzyloxyethyl-2-[4-(4-trifluoromethylphenoxy) phenoxy] propionat erhalten ; $n_D^{20}$ 1,530 0.

### Beispiel 2

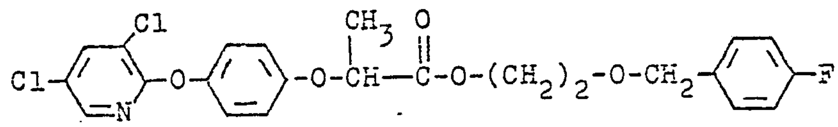

(2)

30,6 g 4-(3,5-Dichloro-2-pyridyloxy) phenol-natriumsalz und 30,5 g 2-(4-Fluorobenzyloxy) ethyl-2-chloropropionat wurden zu 100 ml Dimethylformamid hinzugefügt, und die Mischung wurde 3 h unter Rühren auf 70 °C bis 80 °C erhitzt. Nach dem Abkühlen auf Raumtemperatur wurde die Reaktionslösung in 300 ml Wasser gegossen, und danach wurde mit Ether extrahiert. Die Ether-Schicht wurde nacheinander mit einer wäßrigen Natriumhydroxid-Lösung einer Stärke von 1 Gew.-% und mit Wasser gewaschen und anschließend entwässert. Nach dem Abdestillieren des Ethers wurden als Endprodukt 38 g blaßgelbes viskoses öliges 2-(4-Fluorobenzyloxy) ethyl-2-[4-(3,5-dichloro-2-pyridyloxy) phenoxy] propionat erhalten ; $n_D^{20}$ 1,564 3.

### Beispiel 3

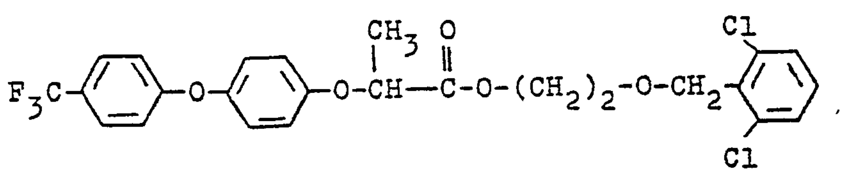

(3)

22,1 g 2-(2,6-Dichlorobenzyloxy) ethanol und 10,1 g Triethylamin wurden in 200 ml Toluol aufgelöst, und die Lösung wurde auf 0 °C abgekühlt. Zu dieser Lösung wurde tropfenweise bei 0 °C bis 10 °C eine Lösung von 34,5 g 2-[4-(4-Trifluoromethylphenoxy) phenoxy] propionylchlorid in 50 ml Toluol hinzugefügt. Nach Beendigung des Zutropfens wurde die Reaktionsmischung allmählich erhitzt und zur Vervollständigung der Reaktion 1 h bei 40 °C gerührt. Nach dem Abkühlen der Reaktionslösung auf Raumtemperatur wurde sie nacheinander mit wäßriger Natriumhydroxid-Lösung einer Stärke von 1 Gew.-% und mit Wasser gewaschen und entwässert. Nach dem Abdestillieren des Toluols unter vermindertem Druck wurde das entstandene farblose viskose ölige Produkt etwa einen Monat stehen gelassen, wonach 49,2 g kristallines 2-(2,6-Dichlorobenzyloxy) ethyl-2-[4-(4-trifluoromethylphenoxy) phenoxy] propionat erhalten wurden ; Schmp. 51-53 °C.

Beispiel 4

20 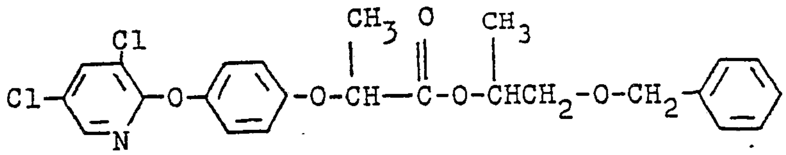 (IV)

16,6 g 1-Benzyloxy-2-propanol und 10,6 g Triethylamin wurden in 200 ml Toluol aufgelöst, und die Lösung wurde auf 0 °C abgekühlt. Zu dieser Lösung wurde tropfenweise unter Rühren bei 0 °C bis 5 °C eine Lösung von 34,7 g 2-[4-(3,5-Dichloro-2-pyridyloxy) phenoxy] propionylchlorid in 80 ml Toluol hinzugefügt. Nach Beendigung des Zutropfens wurde die Mischung allmählich erhitzt und zur Vervollständigung der Reaktion 1 h bei 40 °C bis 50 °C gerührt. Nach dem Abkühlen auf Raumtemperatur wurde sie nacheinander mit wäßriger Natriumhydroxid-Lösung einer Stärke von 1 Gew.-% und mit Wasser gewaschen und danach getrocknet. Nach dem Abdestillieren des Toluols unter vermindertem Druck wurden 43,3 g blaßgelbes viskoses öliges 1-Methyl-2-benzyloxyethyl-2-[4-(3,5-dichloro-2-pyridyloxy) phenoxy] propionat erhalten ; $n_D^{20}$ 1,571 0.

Beispiel 5

In der gleichen Weise wie in Beispiel 1 oder 2 beschrieben, jedoch unter Verwendung der in der nachstehenden Tabelle aufgeführten Ausgangsstoffe, wurden die in der nachstehenden Tabelle angegebenen Verbindungen gemäß der vorliegenden Erfindung synthetisiert.

(Siehe Tabelle Seite 32 ff.)

| Beispiel Nr. | Ausgangsstoff | Ausgangsstoff | Produkt |
|---|---|---|---|
| 6 | 4-(4-Trifluoromethylphenoxy)phenol | 1-Methyl-2-(2-fluorobenzyloxy)ethyl-2-chloropropionat | 1-Methyl-2-(2-fluorobenzyloxy)-ethyl-2-/¯4-(4-trifluoromethylphenoxy)phenoxy_7propionat |
| 7 | " | 3-(2-Fluorobenzyloxy)propyl-2-bromopropionat | 3-(2-Fluorobenzyloxy)propyl-2-/¯4-(4-trifluoromethylphenoxy)-phenoxy_7propionat |
| 8 | 4-(4-Trifluoromethylphenoxy)phenol-natriumsalz | 2-(3-Chlorobenzyloxy)ethyl-2-chloropropionat | 2-(3-Chlorobenzyloxy)ethyl-2-/¯4-(4-trifluoromethylphenoxy)-phenoxy_7propionat |
| 9 | " | 2-(2-Bromobenzyloxy)ethyl-2-chloropropionat | 2-(2-Bromobenzyloxy)ethyl-2-/¯4-(4-trifluoromethylphenoxy)-phenoxy_7propionat |
| 10 | " | 2-(4-Methylbenzyloxy)ethyl-2-bromopropionat | 2-(4-Methylbenzyloxy)ethyl-2-/¯4-(4-trifluoromethylphenoxy)-phenoxy_7propionat |

| Beispiel Nr. | Ausgangsstoff | Ausgangsstoff | Produkt |
|---|---|---|---|
| 11 | 4-(4-Trifluoromethylphenoxy)phenol-natriumsalz | 2-(2-Methoxybenzyloxy)ethyl-2-bromopropionat | 2-(2-Methoxybenzyloxy)-ethyl-2-/¯4-(4-trifluoromethyl-phenoxy)phenoxy_7propionat |
| 12 | 4-(4-Trifluoromethyl-2-chlorophenoxy)phenol-Na-Salz | 2-Benzyloxyethyl-2-chloropropionat | 2-Benzyloxyethyl-2-/¯4-(4-trifluoromethyl-2-chloro-phenoxy)phenoxy_7propionat |
| 13 | 4-(4-Bromo-2-chlorophenoxy)phenol-natriumsalz | " | 2-Benzyloxyethyl-2-/¯4-(4-bromo-2-chlorophenoxy)-phenoxy_7propionat |
| 14 | 4-(2-Chloro-4-cyanophenoxy)phenol-natriumsalz | " | 2-Benzyloxyethyl-2-/¯4-(2-chloro-4-cyanophenoxy)-phenoxy_7propionat |
| 15 | 4-(2,6-Dichloro-4-trifluoromethylphenoxy)phenol-natriumsalz | " | 2-Benzyloxyethyl-2-/¯4-(2,6-dichloro-4-trifluoromethylphenoxy)-phenoxy_7propionat |

| Beispiel Nr. | Ausgangsstoff | Ausgangsstoff | Produkt/Physikalische Konstante |
|---|---|---|---|
| 16 | 4-(3,5-Dichloro-2-pyridyloxy)phenol-natriumsalz | 1-Methyl-2-α-methylbenzyloxyethyl-2-chloropropionat | 1-Methyl-2-α-methylbenzyloxyethyl-2-/‾4-(3,5-dichloro-2-pyridyloxy)phenoxy_7propionat |
| 17 | " | 3-(4-Fluorobenzyloxy)propyl-2-bromopropionat | 3-(4-Fluorobenzyloxy)propyl-2-/‾4-(3,5-dichloro-2-pyridyloxy)-phenoxy_7propionat |
| 18 | " | 2-(3-Chlorobenzyloxy)ethyl-2-chloropropionat | 2-(3-Chlorobenzyloxy)ethyl-2-/‾4-(3,5-dichloro-2-pyridyloxy)-phenoxy_7propionat |
| 19 | " | 2-(4-Methylbenzyloxy)ethyl-2-chloropropionat | 2-(4-Methylbenzyloxy)ethyl-2-/‾4-(3,5-dichloro-2-pyridyloxy)-phenoxy_7propionat |
| 20 | 2-Benzyloxyethanol | 2-/‾4-(5-Trifluoromethyl-2-pyridyloxy)-phenoxy_7propionylchlorid | 2-Benzyloxyethyl-2-/‾4-(5-trifluoromethyl-2-pyridyloxy)phenoxy_7propionat; Schmp. 44-47°C. |

| Beispiel Nr. | Ausgangsstoff | Ausgangsstoff | Produkt/Physikalische Konstante |
|---|---|---|---|
| 21 | 2-(2-Fluoro-benzyloxy)-ethanol | 2-/¯4-(5-Trifluoro-methyl-2-pyridyloxy)-phenoxy_7propionylchlorid | 2-(2-Fluorobenzyloxy)ethyl-2-/¯4-(5-trifluoromethyl-2-pyridyloxy)-phenoxy_7propionat;Schmp.46-48,5°C. |
| 22 | 2-(4-Chloro-benzyloxy)-ethanol | " | 2-(4-Chlorobenzyloxy)ethyl-2-/¯4-(5-trifluoromethyl-2-pyridyloxy)-phenoxy_7propionat; $n_D^{20}$ 1,5350. |
| 23 | 2-(4-Methoxy-benzyloxy)-ethanol | 2-/¯4-(5-Trifluoro-methyl-2-pyridyloxy)-phenoxy_7propionylbromid | 2-(4-Methoxybenzyloxy)ethyl-2-/¯4-(5-trifluoromethyl-2-pyridyloxy)-phenoxy_7propionat; $n_D^{20}$ 1,5335. |
| 24 | 1-Benzyloxy-2-propanol | " | 1-Methyl-2-benzyloxyethyl-2-/¯4-(5-trifluoromethyl-2-pyridyloxy)-phenoxy_7propionat; $n_D^{20}$ 1,5224. |
| 25 | 3-Benzyloxy-propanol | 2-/¯4-(Trifluoro-methyl-2-pyridyloxy)-phenoxy_7propionylchlorid | 3-Benzyloxypropyl-2-/¯4-(5-trifluoromethyl-2-pyridyloxy)phenoxy_7propionat; $n_D^{20}$ 1,5245. |

| Beispiel Nr. | Ausgangsstoff | Ausgangsstoff | Produkt |
|---|---|---|---|
| 26 | 2-Benzyloxyethanol | 2-/¯4-(3-Chloro-5-nitro-2-pyridyloxy)-phenoxy_7propionylbromid | 2-Benzyloxyethyl-2-/¯4-(3-chloro-5-nitro-2-pyridyloxy)phenoxy_7propionat |
| 27 | " | 2-/¯4-(3-Chloro-5-trifluoromethyl-2-pyridyloxy)phenoxy_7propionylchlorid | 2-Benzlyoxyethyl-2-/¯4-(3-chloro-5-trifluoromethyl-2-pyridyloxy)-phenoxy_7propionat |
| 28 | 2-(2-Methoxybenzyloxy)ethanol | 2/¯4-(3,5-Dichloro-2-pyridyloxy)phenoxy_7propionylchlorid | 2-(2-Methoxybenzyloxy)ethyl-2-/¯4-(3,5-dichloro-2-pyridyloxy-phenoxy_7propionat |

Beispiel 29

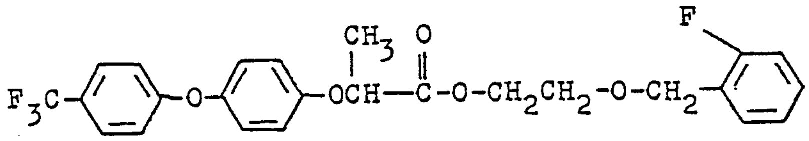 (29)

33,4 g 2-(2-Fluorobenzyloxy) ethyl-2-(4-hydroxyphenoxy)-propionat wurden in 120 ml Dimethylformamid gelöst, und 15,2 g Kaliumcarbonat wurden hinzugegeben ; danach wurde die Mischung unter Rühren 1 h auf 95 °C erhitzt. 27,1 g 4-Trifluoromethylchlorobenzol wurden der Lösung zugesetzt, die danach 5 h auf 95 °C bis 100 °C erhitzt wurde. Nach dem Abkühlen auf Raumtemperatur wurde die Reaktionslösung in Eiswasser gegossen und dann mit Toluol extrahiert. Die Toluol-Schicht wurde nacheinander mit wäßriger Natriumhydroxid-Lösung einer Stärke von 1 Gew.-% und mit Wasser gewaschen und dann getrocknet und filtriert. Das Toluol wurde unter vermindertem Druck abdestilliert, und weitere flüchtige Nebenprodukte wurden bei 100 °C/0,13 mbar (0,1 mm Hg) entfernt. Das Endprodukt 2-(2-Fluorobenzyloxy) ethyl-2-[4-(4-trifluoromethylphenoxy) phenoxy] propionat wurde in einer ausbeute von 20,7 g erhalten ($n_D^{20}$ 1,523 5).

Beispiel 30

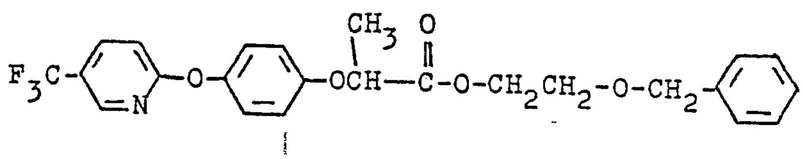 (30)

31,6 g 2-Benzyloxyethyl-2-(4-hydroxyphenoxy)propionat wurden in 100 ml Dimethylsulfoxid aufgelöst, und 15,2 g Kaliumcarbonat wurden hinzugegeben ; danach wurde die Mischung unter Rühren 1 h auf 90 °C erhitzt. 20,0 g 2-Chloro-5-trifluoromethylpyridin wurden tropfenweise zu dieser Lösung hinzugegeben, die dann weitere 2 h auf 90 °C gehalten wurde. Nach dem Abkühlen auf Raumtemperatur wurde die Reaktionslösung in Eiswasser gegossen und dann mit Ether extrahiert. Die Ether-Schicht wurde getrocknet und filtriert, und der Ether wurde abdestilliert. Das Endprodukt 2-Benzyloxyethyl-2-[4-(5-trifluoromethyl-2-pyridyloxy)phenoxy] propionat (eine Verbindung, die auch in Beispiel 20 hergestellt wurde) wurde in einer Ausbeute von 41,0 g erhalten (Schmp. 44-47 °C).

Beispiele 31 bis 79

Die in der nachstehenden Tabelle 5 aufgeführten Verbindungen der vorliegenden Erfindung wurden im wesentlichen in der gleichen Weise wie in den vorhergehenden Beispielen 1 bis 30 (oder, im Falle von Beispiel 40, wie in den vorhergehenden Beispielen 1 bis 28) erhalten.

Tabelle 5

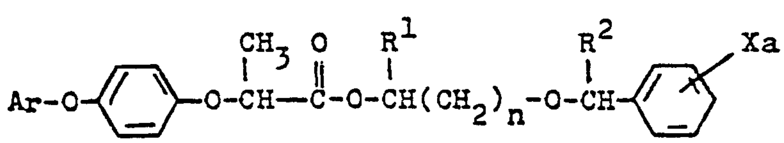

| Beispiel Nr. | Ar | $R^1$ | $R^2$ | n | Xa | Physikal. Konstante |
|---|---|---|---|---|---|---|
| 31 | 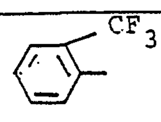 | H | H | 1 | H | $n_D^{20}$ 1,5322 |
| 32 | 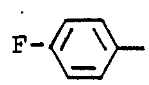 | H | H | 1 | H | $n_D^{20}$ 1,551[illegible] |
| 33 | 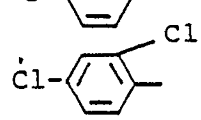 | H | H | 1 | H | $n_D^{20}$ 1,5696 |
| 34 | 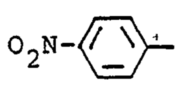 | H | H | 1 | H | $n_D^{20}$ 1,5805 |

Tabelle 5 — Fortsetzung

| Beispiel Nr. | Ar | $R^1$ | $R^2$ | n | Xa | Physikal. Konstante |
|---|---|---|---|---|---|---|
| 35 | 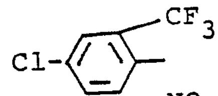 | H | H | 1 | H | $n_D^{20}$ 1,5397 |
| 36 | 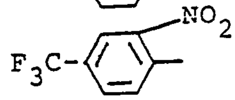 | H | H | 1 | H | $n_D^{20}$ 1,5450 |
| 37 | 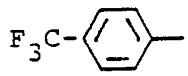 | H | H | 2 | H | $n_D^{20}$ 1,5268 |
| 38 | 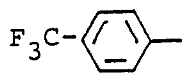 | $-CH_3$ | H | 1 | H | $n_D^{20}$ 1,5260 |
| 39 | 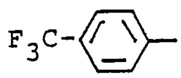 | H | $-CH_3$ | 1 | H | $n_D^{20}$ 1,5266 |
| 40 | 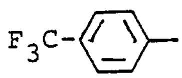 | H | H | 1 | 2-F | $n_D^{20}$ 1,5235 |
| 41 | 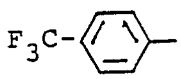 | H | H | 1 | 4-F | $n_D^{20}$ 1,5214 |
| 42 | 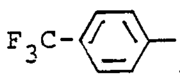 | H | H | 1 | 2-Cl | $n_D^{20}$ 1,5360 |
| 43 | 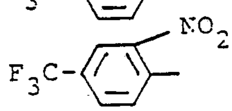 | H | H | 1 | 2-Cl | $n_D^{20}$ 1,5550 |
| 44 | 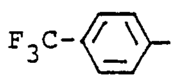 | H | H | 1 | 4-Cl | $n_D^{20}$ 1,5360 |
| 45 | 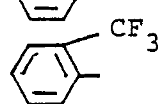 | H | H | 1 | 4-Cl | $n_D^{20}$ 1,5380 |
| 46 | 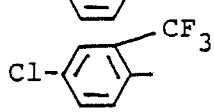 | H | H | 1 | 4-Cl | $n_D^{20}$ 1,5450 |
| 47 | 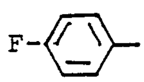 | H | H | 1 | 4-Cl | $n_D^{20}$ 1,5559 |
| 48 | 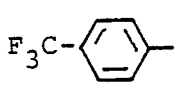 | H | H | 1 | $2,4-Cl_2$ | $n_D^{20}$ 1,5422 |
| 49 | 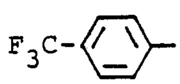 | H | H | 1 | $3,4-Cl_2$ | $n_D^{20}$ 1,5428 |
| 50 | 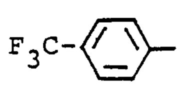 | H | H | 1 | $2-CH_3$ | $n_D^{20}$ 1,5305 |
| 51 | 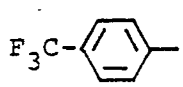 | H | H | 1 | $3-NO_2$ | $n_D^{20}$ 1,5436 |
| 52 | 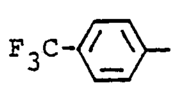 | H | H | 1 | $4-OCH_3$ | $n_D^{20}$ 1,5343 |
| 53 | 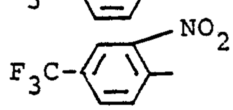 | H | H | 2 | H | $n_D^{20}$ 1,5440 |

Tabelle 5 — Fortsetzung

| Beispiel Nr. | Ar | $R^1$ | $R^2$ | n | Xa | Physikal. Konstante |
|---|---|---|---|---|---|---|
| 54 | 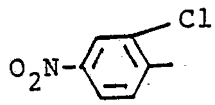 | H | H | 1 | 4-Cl | $n_D^{20}$ 1,5875 |
| 55 | 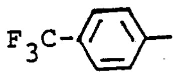 | H | H | 1 | 4-Br | $n_D^{20}$ 1,5450 |
| 56 | 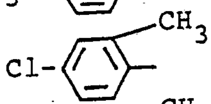 | H | H | 1 | H | $n_D^{20}$ 1,5650 |
| 57 | 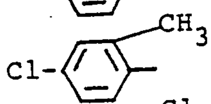 | H | H | 1 | 2-Cl | $n_D^{20}$ 1,5718 |
| 58 | 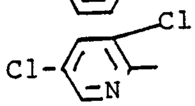 | H | H | 1 | H | Schmp.59-61°C |
| 59 | 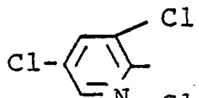 | H | $CH_3$ | 1 | H | $n_D^{20}$ 1,5715 |
| 60 | 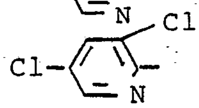 | H | H | 2 | H | $n_D^{20}$ 1,5721 |
| 61 | 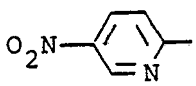 | H | H | 1 | H | Öl |
| 62 | 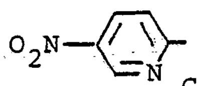 | H | H | 1 | 3-$NO_2$ | Öl |
| 63 | 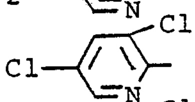 | H | H | 1 | 2-F | Schmp.54-57°C |
| 64 | 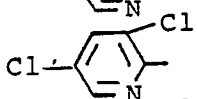 | H | H | 1 | 2-Cl | Schmp.64-66°C |
| 65 | 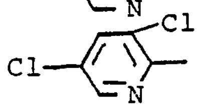 | H | H | 1 | 4-Cl | $n_D^{20}$ 1,5803 |
| 66 | 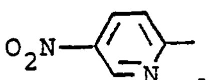 | H | H | 1 | 4-Cl | Öl |
| 67 | 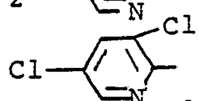 | H | H | 1 | 3-$NO_2$ | $n_D^{20}$ 1,5845 |
| 68 | 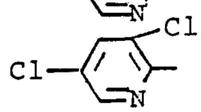 | H | H | 1 | 2-$CH_3$ | Schmp.83-85°C |
| 69 | 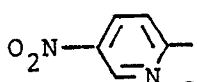 | H | H | 1 | 2,4-$Cl_2$ | Öl |
| 70 | 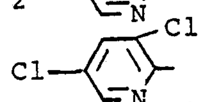 | H | H | 1 | 3,4-$Cl_2$ | $n_D^{20}$ 1,5900 |
| 71 | 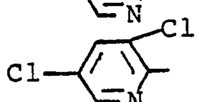 | H | H | 1 | 2,6-$Cl_2$ | $n_D^{20}$ 1,5915 |
| 72 | 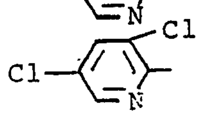 | H | H | 1 | 4-$OCH_3$ | $n_D^{20}$ 1,5735 |

Tabelle 5 — Fortsetzung

| Beispiel Nr. | Ar | $R^1$ | $R^2$ | n | Xa | Physikal. Konstante |
|---|---|---|---|---|---|---|
| 73 | 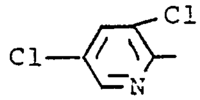 | H | H | 1 | 4-Br | $n_D^{20}$ 1,5870 |
| 74 | 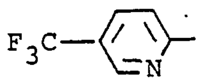 | H | $CH_3$ | 1 | H | $n_D^{20}$ 1,5254 |
| 75 | 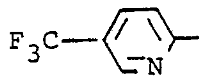 | H | H | 1 | 2-$CH_3$ | $n_D^{20}$ 1,5273 |
| 76 | 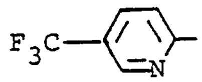 | H | H | 1 | 2-Cl | $n_D^{20}$ 1,5349 |
| 77 | 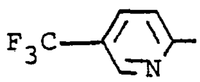 | H | H | 1 | 4-Br | $n_D^{20}$ 1,5440 |
| 78 | 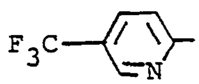 | H | H | 1 | 3-$NO_2$ | $n_D^{20}$ 1,5428 |
| 79 | 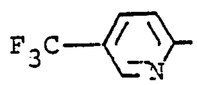 | H | H | 1 | 2,4-$Cl_2$ | $n_D^{20}$ 1,5424 |

Verfahren zur Herstellung der neuen Zwischenprodukt-Verbindungen der Formeln (III) und (VII) gemäß der vorliegenden Erfindung werden anhand der folgenden Beispiele erläutert.

Beispiel 80

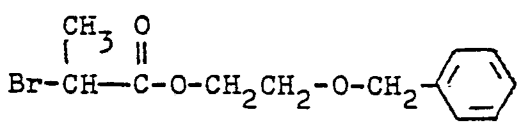

(eine Verbindung der Formel (III))

15,2 g Benzyloxyethanol und 10,6 g Triethylamin wurden in 150 ml Toluol gelöst, und die Lösung wurde auf – 5 °C abgekühlt. Unter Rühren wurde bei – 5 °C bis 0 °C tropfenweise eine Lösung von 21,6 g 2-Bromopropionylbromid in 30 ml Toluol hinzugegeben. Die Reaktionsmischung wurde danach weitere 2 h auf Raumtemperatur gehalten und dann nacheinander mit Natriumhydroxid-Lösung einer Stärke von 1 Gew.-% und mit Wasser gewaschen, und die Toluol-Lösung wurde danach getrocknet und filtriert. Das Toluol wurde unter vermindertem Druck abdestilliert, und das Endprodukt 2-Benzyloxyethyl-2-bromopropionat wurde in einer Ausbeute von 26,7 g erhalten ($n_D^{20,5}$ 1,518 2).

Die Zwischenprodukte der allgemeinen Formel (III) gemäß der vorliegenden Erfindung, die in Tabelle 6 aufgeführt sind, wurden in ähnlicher Weise wie im Vorstehenden beschrieben erhalten.

(Siehe Tabelle 6 Seite 41 f.)

Tabelle 6

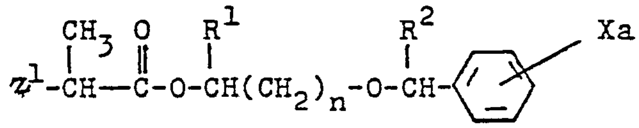

(III)

| $Z^1$ | $R^1$ | $R^2$ | n | $X_a$ | Physikalische Konstante |
|---|---|---|---|---|---|
| Br | H | H | 1 | H | $n_D^{20,5}$ 1,5182 |
| Br | H | H | 1 | 2-F | $n_D^{20}$ 1,5038 |
| Cl | H | H | 1 | 4-F | $n_D^{20}$ 1,5004 |
| Br | H | H | 1 | 4-F | $n_D^{20}$ 1,5040 |
| Br | H | H | 1 | 2-Cl | $n_D^{20,5}$ 1,5302 |
| Br | H | H | 1 | 4-Cl | $n_D^{20}$ 1,5289 |
| Cl | H | H | 1 | 4-Br | $n_D^{20,5}$ 1,5321 |
| Br | H | H | 1 | 4-Br | $n_D^{20,5}$ 1,5434 |
| Br | H | H | 1 | 2-$CH_3$ | $n_D^{20}$ 1,5186 |
| Br | H | H | 1 | 2-$OCH_3$ | $n_D^{20,5}$ 1,5260 |
| Br | H | H | 1 | 3-$NO_2$ | $n_D^{20}$ 1,5394 |
| Br | H | H | 1 | 2,4-$Cl_2$ | $n_D^{20}$ 1,5395 |
| Br | H | H | 1 | 3,4-$Cl_2$ | $n_D^{20}$ 1,5409 |
| Cl | H | H | 1 | 2,6-$Cl_2$ | $n_D^{20}$ 1,5412 |
| Br | $CH_3$ | H | 1 | H | $n_D^{20,5}$ 1,5100 |
| Br | H | $CH_3$ | 1 | H | $n_D^{20,5}$ 1,5116 |
| Br | H | H | 2 | H | $n_D^{20,5}$ 1,5130 |
| Br | H | H | 1 | 4-$OCH_3$ | $n_D^{20,5}$ 1,5250 |

Beispiel 81

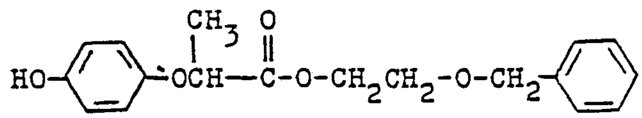

(eine Verbindung der Formel (VII))

12,1 g Hydrochinon wurden in 60 ml trockenem Dimethylformamid gelöst; dazu wurden in einem langsamen Stickstoff-Strom 31,7 g Kaliumcarbonat gegeben, und anschließend wurde die Mischung unter Rühren 1 h auf 90 °C bis 95 °C erhitzt. Nach dem Abkühlen der Mischung auf 60 °C wurden ihr tropfenweise 28,7 g 2-Benzyloxyethyl-2-bromopropionat zugesetzt. Die Reaktionsmischung wurde 2 h auf 90 °C erhitzt. Nach dem Abkühlen der Reaktionsmischung auf Raumtemperatur wurde sie in Eiswasser gegossen, auf pH 7 eingestellt und mit 100 ml Toluol extrahiert. Die Toluol-Schicht wurde getrocknet und filtriert, und danach wurde das Toluol unter vermindertem Druck entfernt. Das farblose ölige Produkt, 2-Benzyloxyethyl-2-(4-hydroxyphenoxy)propionat, wurde in einer Ausbeute von 24,6 g erhalten ($n_D^{21,5}$ 1,539 0).

Die als Zwischenprodukte anfallenden Verbindungen der allgemeinen Formel (VII) gemäß der vorliegenden Erfindung, die in Tabelle 7 aufgeführt sind, wurden in ähnlicher Weise wie im Vorstehenden beschrieben erhalten.

Tabelle 7

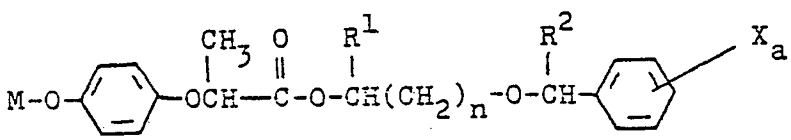

(VII)

| M | $R^1$ | $R^2$ | n | $X_a$ | Physikalische Konstante |
|---|---|---|---|---|---|
| H | H | H | 1 | H | $n_D^{21,5}$ 1,5390 |
| H | H | H | 1 | 2-F | $n_D^{21,5}$ 1,5260 |
| H | H | H | 1 | 4-Cl | $n_D^{21,5}$ 1,5473 |
| H | H | H | 1 | 3,4-$Cl_2$ | $n_D^{21,5}$ 1,5570 |
| H | $CH_3$ | H | 1 | H | $n_D^{20,5}$ 1,5321 |
| H | H | $CH_3$ | 1 | H | $n_D^{20,5}$ 1,5336 |
| H | H | H | 2 | H | $n_D^{21,5}$ 1,5344 |
| H | H | H | 1 | 4-$OCH_3$ | $n_D^{20,5}$ 1,5422 |

**Patentansprüche**

1. Substituierte Phenoxypropionate der allgemeinen Formel

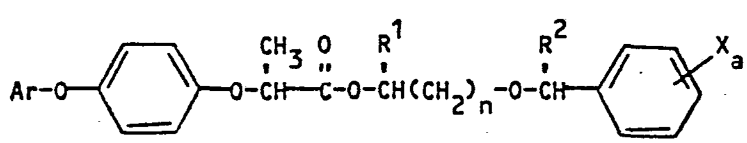

(I)

in der

$R^1$ und $R^2$ jeweils unabhängig voneinander ein Wasserstoff-Atom oder eine $C_1$- bis $C_6$-Alkyl-Gruppe bezeichnen,

X für ein Wasserstoff- oder Halogen-Atom oder eine Nitro-, $C_1$- bis $C_6$-Alkyl- oder $C_1$- bis $C_6$-Alkoxy-Gruppe steht,

a und n jeweils unabhängig voneinander 1 oder 2 sind und

Ar eine Gruppe der allgemeinen Formeln

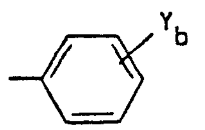
oder
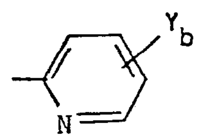

(Ia) (Ib)

bezeichnet, in der

Y für eine Trifluoromethyl-Gruppe, ein Halogen-Atom oder eine Nitro-, Cyano oder $C_1$ bis $C_6$-Alkyl-Gruppe steht und

b 1, 2 oder 3 ist.

2. Substituierte Phenoxypropionate der Formel (I), in denen

$R^1$ und $R^2$ jeweils unabhängig voneinander ein Wasserstoff-Atom oder eine Methyl-, Ethyl-, n-Propyl-, Isopropyl, n-, iso-, sec- oder tert-Butyl-Gruppe bezeichnen ;

X für ein Wasserstoff-, Fluor-, Chlor-, Brom- oder Iod-Atom, eine Nitro-, Methyl-, Methoxy-, Ethyl-, Ethoxy-, n-Propyl-, n-Propoxy-, Isopropyl-, Isopropoxy- oder n-, iso-, sec- oder tert-Butyl oder -Butoxy-Gruppe steht,

a und n jeweils unabhängig voneinander 1 oder 2 sind und

Ar eine Gruppe der Formeln

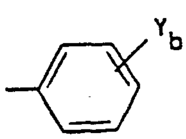
oder
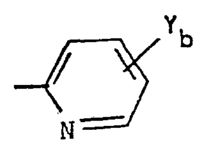

(Ia) (Ib)

bezeichnet,

worin

Y für eine Trifluoromethyl-Gruppe, ein Fluor-, Chlor-, Brom- oder Iod-Atom, oder eine Nitro-, Cyano-, Methyl-, Ethyl-, Propyl-, Isopropyl-, n-, iso-, sec- oder tert-Butyl-Gruppe steht und

b für 1, 2 oder 3 steht.

3. Substituiertes Phenoxypropionat gemäß Anspruch 1, gekennzeichnet durch die Formel

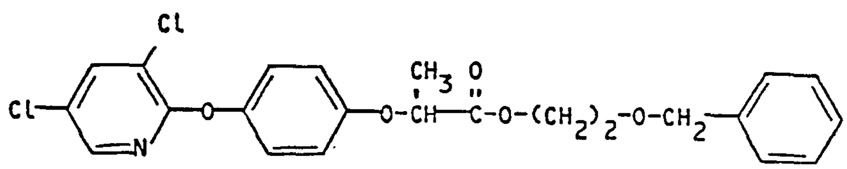

4. Verfahren zur Herstellung von substituierten Phenoxypropionaten der Formel

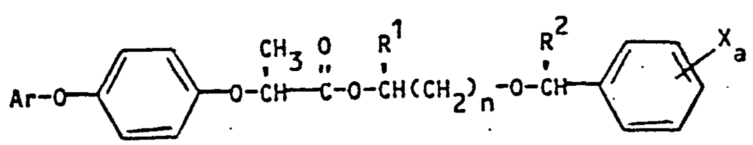
(I)

in der

$R^1$ und $R^2$ jeweils unabhängig voneinander ein Wasserstoff-Atom oder eine $C_1$- bis $C_6$-Alkyl-Gruppe bezeichnen,

X für ein Wasserstoff- oder Halogen-Atom oder eine Nitro-, $C_1$- bis $C_6$-Alkyl- oder $C_1$- bis $C_6$-Alkoxy-Gruppe steht,

a und n jeweils unabhängig voneinander 1 oder 2 sind und

Ar eine Gruppe der allgemeinen Formeln

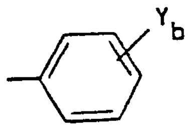

oder

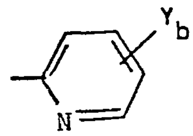

(Ia) (Ib)

bezeichnet, in der

Y für eine Trifluoromethyl-Gruppe, ein Halogen-Atom oder eine Nitro-, Cyano- oder $C_1$- bis $C_6$-Alkyl-Gruppe steht und

b 1, 2 oder 3 ist,

dadurch gekennzeichnet, daß man

a) eine Verbindung der Formel

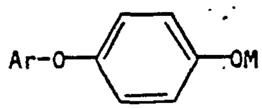

(II)

in der

Ar die oben angegebene Bedeutung hat und

M ein Wasserstoff-Atom oder ein Alkalimetall-Atom bezeichnet,

mit einer Verbindung der allgemeinen Formel

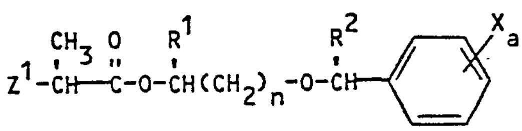

(III)

in der

$R^1$, $R^2$, X, a und n die oben angegebenen Bedeutungen haben und

$Z^1$ für ein Halogen-Atom steht,

gegebenenfalls in Gegenwart eines Säurebindemittels und gegebenenfalls in Gegenwart eines Verdünnungsmittels umsetzt,

oder

b) eine Verbindung der allgemeinen Formel

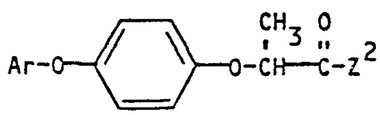

(IV)

in der

Ar die oben angegebene Bedeutung hat und

$Z^2$ für eine Hydroxyl-Gruppe oder ein Halogen-Atom steht,

mit einer Verbindung der allgemeinen Formel

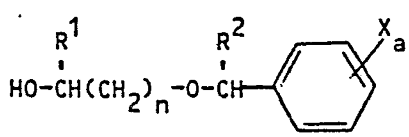

(V)

in der $R^1$, $R^2$, X, a und n die oben angegebenen Bedeutungen haben,

gegebenenfalls in Gegenwart eines Säurebindemittels und gegebenenfalls in Gegenwart eines Verdünnungsmittels umsetzt,

oder

c) eine Verbindung der allgemeinen Formel

$$Ar—Z^1 \qquad (VI)$$

in der Ar und $Z^1$ die oben angegebenen Bedeutungen haben,

mit einer Verbindung der allgemeinen Formel

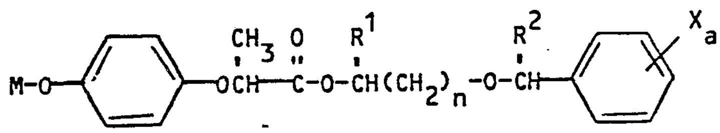

(VII)

in der

$R^1$, $R^2$, X, a und n die oben angegebenen Bedeutungen haben und

M für ein Wasserstoff- oder Alkalimetall-Atom steht,

gegebenenfalls in Gegenwart eines Säurebindemittels sowie gegebenenfalls in Gegenwart eines Verdünnungsmittels umsetzt.

5. Herbizide Mittel, gekennzeichnet durch einen Gehalt an mindestens einem substituierten Phenoxypropionat der Formel (I).

6. Verwendung von substituierten Phenoxypropionaten der Formel (I) zur Bekämpfung von Unkräutern.

7. Substituierte 2-Halogenopropionate der allgemeinen Formel

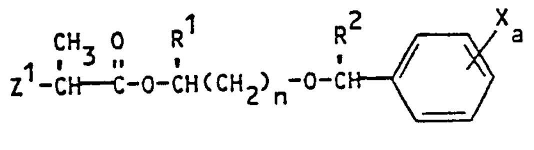

(III)

in der

$R^1$ und $R^2$ jeweils unabhängig voneinander ein Wasserstoff-Atom oder eine $C_1$- bis $C_6$-Alkyl-Gruppe bezeichnen,

X für ein Wasserstoff- oder Halogen-Atom oder eine Nitro-, $C_1$- bis $C_6$-Alkyl- oder $C_1$- bis $C_6$-Alkoxy-Gruppe steht,

a und n jeweils unabhängig voneinander 1 oder 2 sind und

$Z^1$ ein Halogen-Atom bezeichnet.

8. Verfahren zur Herstellung substituierter 2-Halogenpropionate der Formel

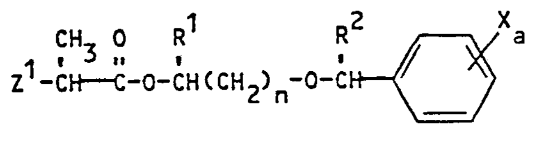

(III)

in der

$R^1$ und $R^2$ jeweils unabhängig voneinander ein Wasserstoff-Atom oder eine $C_1$- bis $C_6$-Alkyl-Gruppe bezeichnen,

X für ein Wasserstoff- oder Halogen-Atom oder eine Nitro-, $C_1$- bis $C_6$-Alkyl- oder $C_1$- bis $C_6$-Alkoxy-Gruppe steht,

a und n jeweils unabhängig voneinander 1 oder 2 sind und

$Z^1$ ein Halogen-Atom bezeichnet,

dadurch gekennzeichnet, daß man ein 2-Halogenopropionylhalogenid der Formel

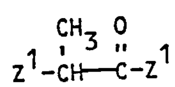

(VIII)

in der $Z^1$ die oben angegebene Bedeutung hat,

mit einer Verbindung der Formel

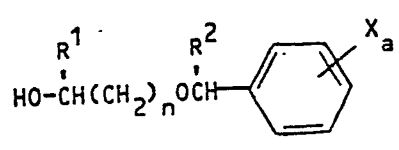

(IX)

in der $R^1$, $R^2$, X, a und n die oben angegebenen Bedeutungen haben,

gegebenenfalls in Gegenwart eines inerten Lösungsmittels und/oder eines säurebindenden Mittels umsetzt.

9. Substituierte 2-(4-Hydroxyphenoxy)-propionate der allgemeinen Formel

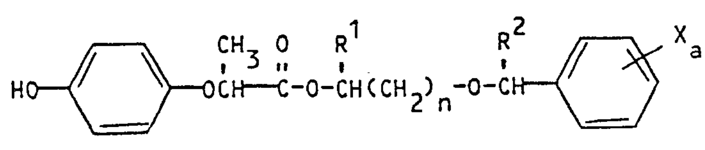

(VII)

in der
$R^1$ und $R^2$ jeweils unabhängig voneinander ein Wasserstoff-Atom oder eine $C_1$- bis $C_6$-Alkyl-Gruppe bezeichnen,
X für ein Wasserstoff- oder Halogen-Atom oder eine Nitro-, $C_1$- bis $C_6$-Alkyl- oder $C_1$- bis $C_6$-Alkoxy-Gruppe steht und
a und n jeweils unabhängig voneinander 1 oder 2 sind.

10. Verfahren zur Herstellung von substituierten 2-(4-Hydroxyphenoxy)-propionaten der Formel

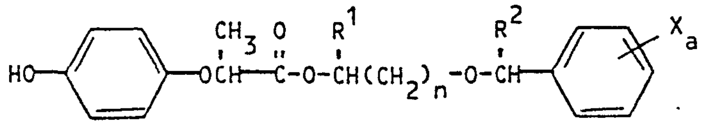

(VII)

in der
$R^1$ und $R^2$ jeweils unabhängig voneinander ein Wasserstoff-Atom oder eine $C_1$- bis $C_6$-Alkyl-Gruppe bezeichnen,
X für ein Wasserstoff- oder Halogen-Atom oder eine Nitro-, $C_1$- bis $C_6$-Alkyl- oder $C_1$- bis $C_6$-Alkoxy-Gruppe steht und
a und n jeweils unabhängig voneinander 1 oder 2 sind,
dadurch gekennzeichnet, daß man Hydrochinon der Formel

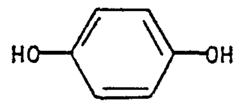

mit einem substituierten 2-Halogenopropionat der Formel

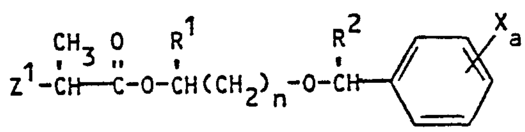

(III)

in der
$R^1$, $R^2$, X, a und n die oben angegebenen Bedeutungen haben und
$Z^1$ ein Halogen-Atom bezeichnet,
gegebenenfalls in Gegenwart eines inerten Lösungsmittels und/oder eines säurebindenden Mittels umsetzt.

## Claims

1. Substituted phenoxypropionates of the general formula

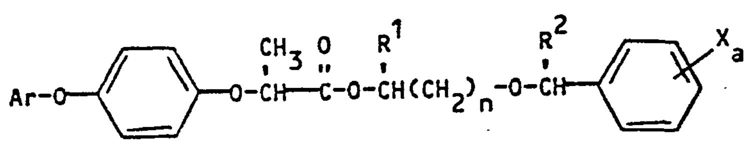

(I)

in which
$R^1$ and $R^2$ each independently of one another designate a hydrogen atom or a $C_1$ to $C_6$ alkyl group,
X represents a hydrogen or halogen atom or a nitro, $C_1$ to $C_6$ alkyl or $C_1$ to $C_6$ alkoxy group,
a and n each independently of one another are 1 or 2 and
Ar designates a group of the general formulae

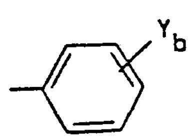

(Ia)

or

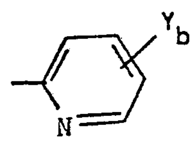

(Ib)

in which
Y represents a trifluoromethyl group, a halogen atom or a nitro, cyano or $C_1$ to $C_6$ alkyl group and
b is 1, 2 or 3.

2. Substituted phenoxypropionates of the formula (I), in which

$R^1$ and $R^2$ each independently of one another designate a hydrogen atom or a methyl, ethyl, n-propyl, isopropyl, n-, iso-, sec- or tert-butyl group,

X represents a hydrogen, fluorine, chlorine, bromine or iodine atom, a nitro, methyl, methoxy, ethyl, ethoxy, n-propyl, n-propoxy, isopropyl, isopropoxy or n-, iso-, sec- or tert-butyl or -butoxy group,

a and n each independently of one another are 1 or 2 and

Ar designates a group of the formulae

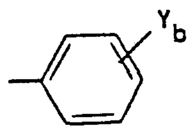

or

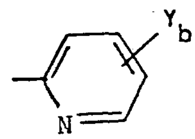

(Ia) (Ib)

wherein

Y represents a trifluoromethyl group, a fluorine, chlorine, bromine or iodine atom, or a nitro, cyano, methyl, ethyl, propyl, isopropyl, n-, iso-, sec- or tert-butyl group and

b represents 1, 2 or 3.

3. Substituted phenoxypropionate according to Claim 1, characterised by the formula

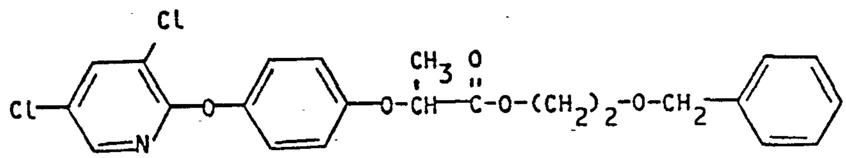

4. Process for the preparation of substituted phenoxypropionates of the formula

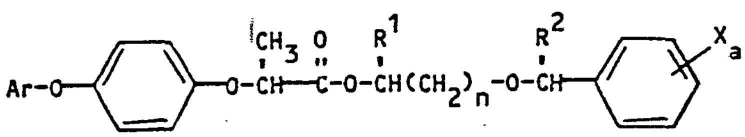

(I)

in which

$R^1$ and $R^2$ each independently of one another designate a hydrogen atom or a $C_1$ to $C_6$ alkyl group,

X represents a hydrogen or halogen atom or a nitro, $C_1$ to $C_6$ alkyl or $C_1$ to $C_6$ alkoxy group,

a and n each independently of one another are 1 or 2 and

Ar designates a group of the general formulae

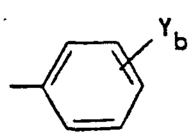

or

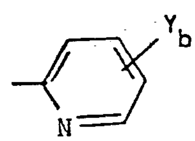

(Ia) (Ib)

in which

Y represents a trifluoromethyl group, a halogen atom or a nitro, cyano or $C_1$ to $C_6$ alkyl group and

b is 1, 2 or 3,

characterised in that

a) a compound of the formula

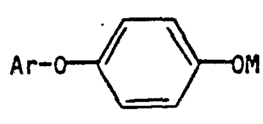

(II)

in which

Ar has the abovementioned meaning and

M designates a hydrogen atom or an alkali metal atom, is reacted with a compound of the general formula

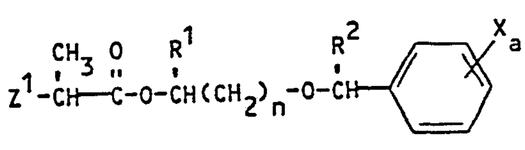

(III)

in which

$R^1$, $R^2$, X, a und n have the abovementioned meanings and

$Z^1$ represents a halogen atom,

if appropriate in the presence of an acid-binding agent and if appropriate in the presence of a diluent, or

b) a compound of the general formula

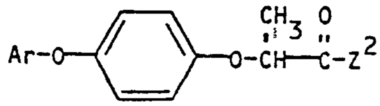

(IV)

in which

Ar has the abovementioned meaning and

$Z^2$ represents a hydroxyl group or a halogen atom,

is reacted with a compound of the general formula

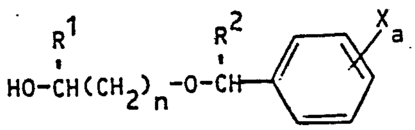

(V)

in which $R^1$, $R^2$, X, a and n have the abovementioned meanings, if appropriate in the presence of an acid-binding agent and if appropriate in the presence of a diluent,

or

c) a compound of the general formula

$$Ar—Z^1 \quad (VI)$$

in which

Ar and $Z^1$ have the abovementioned meanings,

is reacted with a compound of the general formula

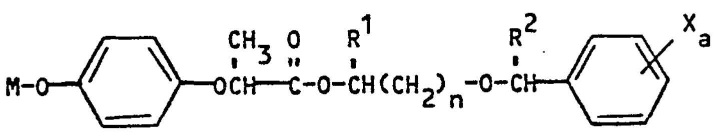

(VII)

in which

$R^1$, $R^2$, X, a and n have the abovementioned meanings and

M represents a hydrogen or alkali metal atom,

if appropriate in the presence of an acid-binding agent and if appropriate in the presence of a diluent.

5. Herbicidal agents, characterised in that they contain at least one substituted phenoxypropionate of the formula (I).

6. Use of substituted phenoxypropionates of the formula (I) for combating weeds.

7. Substituted 2-halogenopropionates of the general formula

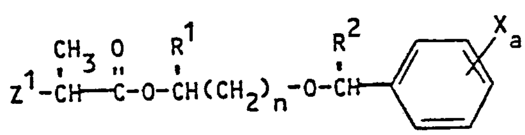

(III)

in which

$R^1$ and $R^2$ each independently of one another designate a hydrogen atom or a $C_1$ to $C_6$ alkyl group,

X represents a hydrogen or halogen atom or a nitro, $C_1$ to $C_6$ alkyl or $C_1$ to $C_6$ alkoxy group,

a and n each independently of one another are 1 or 2 and

$Z^1$ designates a halogen atom.

8. Process for the preparation of substituted 2-halogenopropionates of the formula

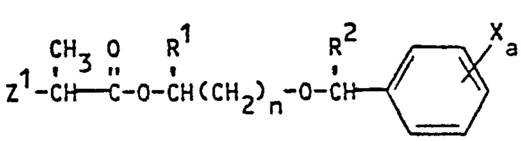

(III)

in which

$R^1$ and $R^2$ each independently of one another designate a hydrogen atom or a $C_1$ to $C_6$ alkyl group,

X represents a hydrogen or halogen atom or a nitro, $C_1$ to $C_6$ alkyl or $C_1$ to $C_6$ alkoxy group,
a and n each independently of one another are 1 or 2 and
$Z_1$ designates a halogen atom,
characterised in that a 2-halogenopropionyl halide of the formula

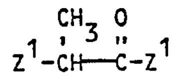

(VIII)

in which $Z^1$ has the abovementioned meaning, is reacted with a compound of the formula

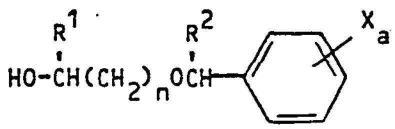

(IX)

in which $R^1$, $R^2$, X, a and n have the abovementioned meanings, if appropriate in the presence of an inert solvent and/or an acid-binding agent.

9. Substituted 2-(4-hydroxyphenoxy)-propionates of the general formula

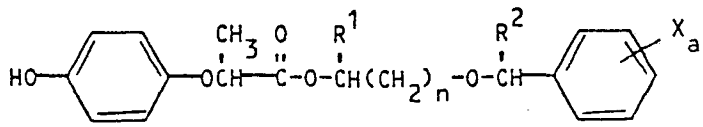

(VII)

in which
$R^1$ and $R^2$ each independently of one another designate a hydrogen atom or a $C_1$ to $C_6$ alkyl group,
X represents a hydrogen or halogen atom or a nitro, $C_1$ to $C_6$ alkyl or $C_1$ to $C_6$ alkoxy group and
a and n each independently of one another are 1 or 2.

10. Process for the preparation of substituted 2-(4-hydroxyphenoxy)-propionates of the formula

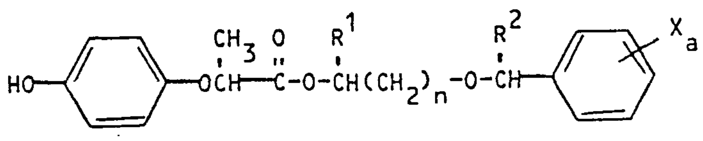

(VII)

in which
$R^1$ and $R^2$ each independently of one another designate a hydrogen atom or a $C_1$ to $C_6$ alkyl group,
X represents a hydrogen or halogen atom or a nitro, $C_1$ to $C_6$ alkyl or $C_1$ to $C_6$ alkoxy group and
a and n each independently of one another are 1 or 2,
characterised in that hydroquinone of the formula

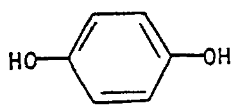

is reacted with a substituted 2-halogenopropionate of the formula

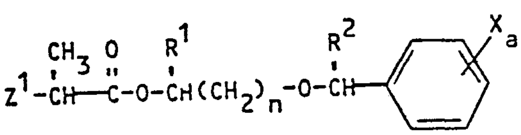

(III)

in which
$R^1$, $R^2$, X, a and n have the abovementioned meanings and
$Z^1$ designates a halogen atom,
if appropriate in the presence of an inert solvent and/or an acid-binding agent.

**Revendications**

1. Phénoxypropionates substitués de formule générale

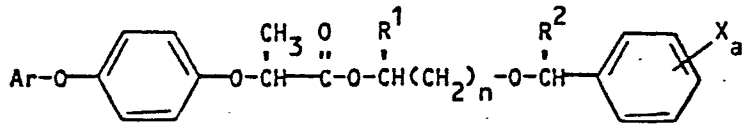 (I)

dans laquelle

$R^1$ et $R^2$ représentent chacun, indépendamment l'un de l'autre, un atome d'hydrogène ou un groupe alkyle en $C_1$-$C_6$,

X représente un atome d'hydrogène ou d'halogène ou un groupe nitro, alkyle en $C_1$-$C_6$ ou alcoxy en $C_1$-$C_6$,

a et n représentent chacun, indépendamment l'un de l'autre, 1 ou 2, et

Ar représente un groupe de formule générale

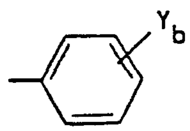 ou 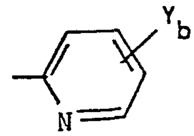

(Ia) (Ib)

dans lequel

Y représente un groupe trifluorométhyle, un atome d'halogène ou un groupe nitro, cyano ou alkyle en $C_1$-$C_6$ et

b est égal à 1, 2 ou 3.

2. Phénoxypropionates substitués de formule I selon la revendication 1, dans lesquels $R^1$ et $R^2$ représentent chacun, indépendamment l'un de l'autre, un atome d'hydrogène ou un groupe méthyle, éthyle, n-propyle, isopropyle, n-, iso-, sec-, tert-butyle,

X représente un atome d'hydrogène, de fluor, de chlore, de brome ou d'iode, un groupe nitro, méthyle, méthoxy, éthyle, éthoxy, n-propyle, n-propoxy, iso-propyle, iso-propoxy ou n-, iso-, sec- ou tert-butyle ou -butoxy,

a et n représentent chacun, indépendamment l'un de l'autre, 1 ou 2, et

Ar représente un groupe de formule

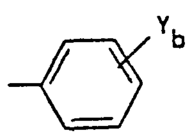 ou 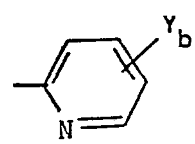

(Ia) (Ib)

dans lequel Y représente un groupe trifluorométhyle, un atome de fluor, de chlore, de brome ou d'iode ou un groupe nitro, cyano, méthyle, éthyle, propyle, iso-propyle, n-, iso-, sec- ou tert-butyle et

b est égal à 1, 2 ou 3.

3. Phénoxypropionate substitué selon la revendication 1, caractérisé par la formule

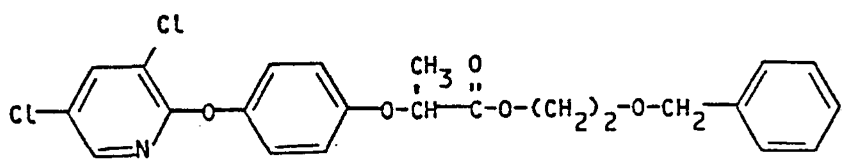

4. Procédé de préparation des phénoxypropionates substitués de formule

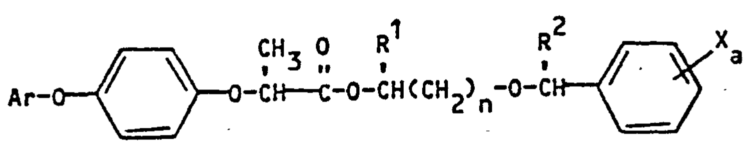 (I)

dans laquelle

$R^1$ et $R^2$ représentent chacun, indépendamment l'un de l'autre, un atome d'hydrogène ou un groupe alkyle en $C_1$-$C_6$,

X représente un atome d'hydrogène ou d'halogène ou un groupe nitro, alkyle en $C_1$-$C_6$ ou alcoxy en $C_1$-$C_6$,

a et n représentent chacun, indépendamment l'un de l'autre, 1 ou 2, et

Ar représente un groupe de formule générale

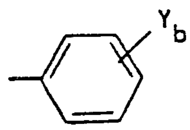

ou

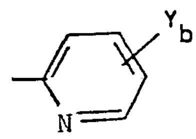

(Ia) (Ib)

dans lequel

Y représente un groupe trifluorométhyle, un atome d'halogène ou un groupe nitro, cyano ou alkyle en $C_1$-$C_6$, et

b est égal à 1, 2 ou 3,

caractérisé en ce que

a) on fait réagir un composé de formule

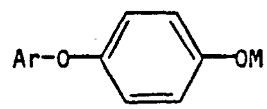

(II)

dans laquelle

Ar a les significations indiquées ci-dessus, et

M représente un atome d'hydrogène ou de métal alcalin, avec un composé de formule générale

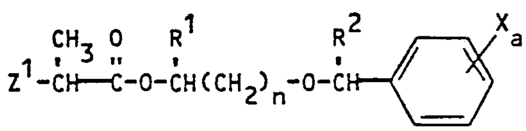

(III)

dans laquelle

$R^1$, $R^2$, X, a et n ont les significations indiquées ci-dessus, et

$Z^1$ représente un atome d'halogène,

éventuellement en présence d'un agent fixant les acides et éventuellement en présence d'un diluant, ou bien

b) on fait réagir un composé de formule générale

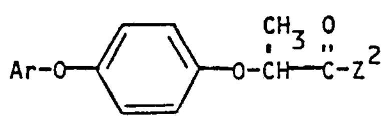

(IV)

dans laquelle

Ar a les significations indiquées ci-dessus, et

$Z^2$ représente un groupe hydroxy ou un atome d'halogène,

avec un composé de formule générale

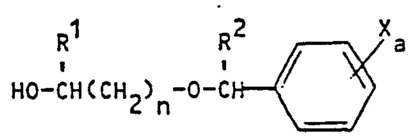

(V)

dans laquelle

$R^1$, $R^2$, X, a et n ont les significations indiquées ci-dessus, éventuellement en présence d'un agent fixant les acides et éventuellement en présence d'un diluant,

ou bien

c) on fait réagir un composé de formule générale

$$Ar—Z^1 \qquad (VI)$$

dans laquelle Ar et $Z^1$ ont les significations indiquées ci-dessus, avec un composé de formule générale

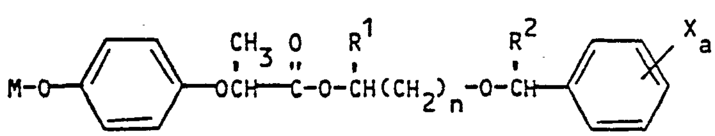

(VII)

dans laquelle

$R^1$, $R^2$, X, a et n ont les significations indiquées ci-dessus, et

M représente un atome d'hydrogène ou de métal alcalin, éventuellement en présence d'un agent fixant les acides et éventuellement en présence d'un diluant.

5. Produit herbicide, caractérisé en ce qu'il contient au moins un phénoxypropionate substitué de formule I.

6. Utilisation des phénoxypropionates substitués de formule I dans la lutte contre les mauvaises herbes.

7. 2-halogénopropionates substitués de formule générale :

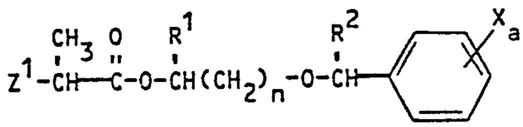

(III)

dans laquelle

$R^1$ et $R^2$ représentent chacun, indépendamment l'un de l'autre, un atome d'hydrogène ou un groupe alkyle en $C_1$-$C_6$,

X représente un atome d'hydrogène ou d'halogène ou un groupe nitro, alkyle en $C_1$-$C_6$ ou alcoxy en $C_1$-$C_6$,

a et n représentent chacun, indépendamment l'un de l'autre, 1 ou 2, et

$Z^1$ représente un atome d'halogène.

8. Procédé de préparation des 2-halogénopropionates substitués de formule

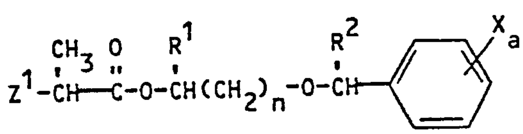

(III)

dans laquelle

$R^1$ et $R^2$ représentent chacun, indépendamment l'un de l'autre, un atome d'hydrogène ou un groupe alkyle en $C_1$-$C_6$,

X représente un atome d'hydrogène ou d'halogène ou un groupe nitro, alkyle en $C_1$-$C_6$ ou alcoxy en $C_1$-$C_6$,

a et n représentent chacun, indépendamment l'un de l'autre, 1 ou 2, et

$Z^1$ représente un atome d'halogène,

caractérisé en ce que l'on fait réagir un halogénure de 2-halogénopropionyle de formule

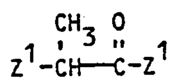

(VIII)

dans laquelle $Z^1$ a les significations indiquées ci-dessus,

avec un composé de formule

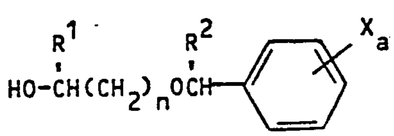

(IX)

dans laquelle

$R^1$, $R^2$, X, a et n ont les significations indiquées ci-dessus, éventuellement en présence d'un solvant inerte et/ou d'un agent fixant les acides.

9. 2-(4-hydroxyphénoxy)-propionates substitués de formule générale

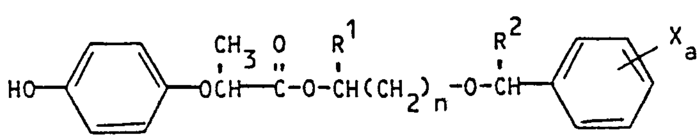

(VII)

dans laquelle

$R^1$ et $R^2$ représentent chacun, indépendamment l'un de l'autre, un atome d'hydrogène ou un groupe alkyle en $C_1$-$C_6$,

X représente un atome d'hydrogène ou d'halogène ou un groupe nitro, alkyle en $C_1$-$C_6$ ou alcoxy en $C_1$-$C_6$, et

a et n représentent chacun, indépendamment l'un de l'autre, 1 ou 2.

10. Procédé de préparation des 2-(4-hydroxyphénoxy)-propionates substitués de formule

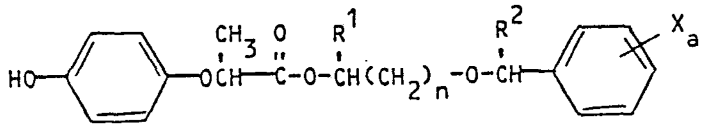

(VII)

dans laquelle

$R^1$ et $R^2$ représentent chacun, indépendamment l'un de l'autre, un atome d'hydrogène ou un groupe alkyle en $C_1$-$C_6$,

X représente un atome d'hydrogène ou d'halogène ou un groupe nitro, alkyle en $C_1$-$C_6$ ou alcoxy en $C_1$-$C_6$, et

a et n représentent chacun, indépendamment l'un de l'autre, 1 ou 2,

caractérisé en ce que l'on fait réagir l'hydroquinone de formule

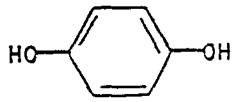

avec un 2-halogénopropionate substitué de formule

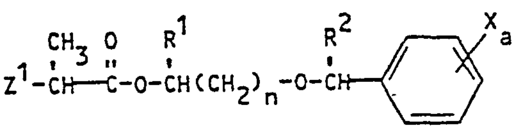

(III)

dans laquelle

$R^1$, $R^2$, X, a et n ont les significations indiquées ci-dessus, et

$Z^1$ représente un atome d'halogène,

éventuellement en présence d'un solvant inerte et/ou d'un agent fixant les acides.